(19) Europäisches Patentamt
European Patent Office
Office européen des brevets

(11) **EP 4 674 344 A1**

(12) **EUROPEAN PATENT APPLICATION**

(43) Date of publication:
07.01.2026 Bulletin 2026/02

(21) Application number: 24185753.1

(22) Date of filing: 01.07.2024

(51) International Patent Classification (IPC):
*A61B 5/08* (2006.01)   *A61B 7/00* (2006.01)

(52) Cooperative Patent Classification (CPC):
A61B 5/0823; A61B 7/003

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: Carepath Technologies GmbH
10115 Berlin (DE)

(72) Inventors:
• **Pant, Satish**
  **10115 Berlin (DE)**
• **Ponti, Federico**
  **10115 Berlin (DE)**

(74) Representative: **Stellbrink & Partner**
**Patentanwälte mbB**
**Widenmayerstrasse 10**
**80538 München (DE)**

(54) **TARGETED USER COUGH DETECTION**

(57)    The present invention relates to a method to detect cough of a targeted user. The method comprises processing a cough audio clip with a targeted cough detector and generating based thereon a result indicative of whether the cough audio clip contains a cough sound of the targeted user. There present invention also relates to a corresponding system.

**Fig. 1**

**EP 4 674 344 A1**

**Description**

The present invention relates to detecting cough and in particular detecting cough of a targeted user.

Cough monitoring has demonstrated clinical significance as it plays a crucial role in tracking health status over time.

Ideally, patients should be monitored in their natural environments using unobtrusive methods. Audio sensing offers a convenient approach to detect cough utterances, which can be analyzed for quantity (number of coughs) or quality (e.g., character: dry, wet cough). This monitoring can aid in understanding health triggers or alerting users or caregivers for early warnings and interventions.

However, unobtrusive monitoring using remote sensing means observing other people's coughs as well.

US 2022/0130415 A1 describes a method of detecting a cough in an audio stream that includes a step of performing one or more pre-processing steps on the audio stream to generate an input audio sequence comprising a plurality of time-separated audio segments. An embedding is generated by a self-supervised triplet loss embedding model for each of the segments of the input audio sequence using an audio feature set, the embedding model having been trained to learn the audio feature set in a self-supervised triplet loss manner from a plurality of speech audio clips from a speech dataset. The embedding for each of the segments is provided to a model performing cough detection inference. This model generates a probability that each of the segments of the input audio sequence includes a cough episode. The method includes generating cough metrics for each of the cough episodes detected in the input audio sequence.

US 2022/0409089 A1 describes a cough detection system and method that uses a first database of physiological information relating to a user for whom cough detection is to be implemented and relating to other people likely to be in the vicinity of the user. A second database (used in real time or as a part of a system calibration) has cough data associated with the physiological information. There is a set of cough detection algorithms, each one tailored to a particular set of physiological characteristics. A cough detection algorithm is selected or constructed which is suitable for identifying coughs of the user while ignoring coughs of the other people. This selected algorithm is applied to sound collected to identify coughs of the user.

Existing machine learning technologies primarily use only a speech dataset for training, which can be limiting for cough detection because the coughs could have absent, short or different vocal component. Hence, the feature engineering for speech might not be optimal for cough detection tasks. This makes it difficult to capture coughs with limited vocal information or speech characteristics.

Moreover, in existing technologies, such as the one described in US 2022/0130415 A1, the output of a model for detecting audio features is typically provided to a cough detector. However, this may create dependency between the cough detector and the self-supervised model. If there is any change in the self-supervised model, then the nature of its output changes. Hence, all downstream modules need to be adapted or changed for proper functioning of the system. That is, whenever there is a change in the self-supervised model, the cough detector model also needs to be changed or retrained. Furthermore, existing technologies provide little flexibility in particular when it comes to downstream tasks having totally different feature engineering requirements compared to initial (upstream) models.

Furthermore, technologies that use triplet loss function face significant challenges in converging during training. This for example is shown in C. -Y. Wu, R. Manmatha, A. J. Smola and P. Krähenbühl, "Sampling Matters in Deep Embedding Learning," 2017 IEEE International Conference on Computer Vision (ICCV), Venice, Italy, 2017, pp. 2859-2867, doi: 10.1109/ICCV.2017.309 and F. Schroff, D. Kalenichenko and J. Philbin, "FaceNet: A unified embedding for face recognition and clustering," 2015 IEEE Conference on Computer Vision and Pattern Recognition (CVPR), Boston, MA, USA, 2015, pp. 815-823, doi: 10.1109/CVPR.2015.7298682. The problem may grow even further when training with large dataset, wherein it may take many practical considerations to train the model. Examples of problems include model collapse during training and high computational complexity.

The present invention alleviates at least some of these shortcomings.

The present invention relates to a method to detect cough of a targeted user. The method comprises processing a cough audio clip with a targeted cough detector and generating based thereon a result indicative of whether the cough audio clip contains a cough sound of the targeted user.

The present invention may thus differentiate cough of the targeted user from other sounds. For example, in one or more audio recordings, cough of the targeted user may be isolated. This may aid, in particular healthcare providers, in tracking cough of a particular user (i.e., the targeted user). For example, the present invention facilitates collecting cough metrics for a particular user which may be used to analyses the cough behavior of the targeted user, such as, detect any change in cough pattern/frequency. The present invention can also be useful in identifying various respiratory diseases.

The targeted cough detector may comprise a processing unit which may be singular or plural, and may be, but not limited to, a CPU (central processing unit), GPU (graphical processing unit), DSP (digital signal processor), APU (accelerator processing unit), ASIC (application-specific integrated circuit), ASIP (application-specific instruction-set processor) or FPGA (field programable gate array). The processing unit may comprise one or more micro-controller units.

Further, the targeted cough detector may comprise a memory component which may be singular or plural, and may be, but is not limited to, a volatile or non-volatile memory, such as a random-access memory (RAM), Dynamic RAM (DRAM), Synchronous Dynamic RAM (SDRAM), static RAM (SRAM), Flash Memory, Magneto-resistive RAM (MRAM), Ferroelectric RAM (F-RAM), or Parameter RAM (P-RAM).

The targeted cough detector may further comprise input and output interfaces for exchanging data electronically.

The method can comprise obtaining source audio data, wherein the source audio data can comprise the cough audio clip. The source audio data may for example comprise audio recordings taken at the vicinity of the targeted user. For example, the source audio data may be recorded in an environment wherein the user is located.

Obtaining the source data can comprise generating the source audio data with an audio recorder.

The method can comprise the audio recorder continuously recording the source audio data.

The audio recorder may be a portable device, a wearable device or a stationary device. This may facilitate recording the source audio data at the vicinity of the targeted user, thus increasing the likelihood that the targeted user coughing may actually be captured in the source audio data.

The audio recorder can comprise at least one auxiliary sensor configured to sense auxiliary data about a surrounding of the at least one auxiliary sensor.

The auxiliary data may be indicative of a humidity, pollution and/or presence of allergens in the surrounding of the at least one auxiliary sensor. This may facilitate explaining the user's coughing behavior.

The method can comprise pre-processing the source audio data with a cough detector to obtain cough audio data, wherein the cough audio data can comprise the cough audio clip. That is, initially all cough sounds, irrespective of whether they correspond to the targeted user or not, may be detected. This may make it easier and more accurate to then detect cough of the targeted user.

The cough detector may comprise a processing unit which may be singular or plural, and may be, but not limited to, a CPU (central processing unit), GPU (graphical processing unit), DSP (digital signal processor), APU (accelerator processing unit), ASIC (application-specific integrated circuit), ASIP (application-specific instruction-set processor) or FPGA (field programable gate array). The processing unit may comprise one or more micro-controller units.

Further, the cough detector may comprise a memory component which may be singular or plural, and may be, but is not limited to, a volatile or non-volatile memory, such as a random-access memory (RAM), Dynamic RAM (DRAM), Synchronous Dynamic RAM (SDRAM), static RAM (SRAM), Flash Memory, Magneto-resistive RAM (MRAM), Ferroelectric RAM (F-RAM), or Parameter RAM (P-RAM).

The cough detector may further comprise input and output interfaces for exchanging data electronically.

The pre-processing may be performed such that the cough audio data contain less non-cough sounds than the source audio data.

The cough detector may be configured to detect cough sounds. For example, the cough detect may search for cough sounds in the source audio data.

The cough detector may be configured to detect non-cough sounds, preferably a plurality of non-cough sound types, more preferably non-cough sound types, wherein each corresponds to a respective sound producible by a human. Detecting non-cough sounds may be advantageous as it may allow filtering them out. Moreover, detecting non-cough sound types producible by a human may be particularly advantageous for privacy reasons. For example, speech, which is an example of a non-cough sound type producible by a human, may be filtered out.

The source audio data may be partitioned in audio segments. This can be advantageous as the source audio data may comprise various lengths and may sometimes be long. By partitioning the source audio data in audio segments, the pre-processing may be facilitated, standardized, and may not depend on the length of the source audio data.

The audio segments may overlap. This may allow for a more thorough pre-processing and may increase the likelihood that cough sounds are detected and/or non-cough sounds are filtered out.

The audio segments can comprise a constant duration, preferably at least 0.5 seconds and at most 5 seconds, more preferably at least 1 second and at most 2 seconds. It will be understood, that while the given durations for the audio segments were found to be advantageous, other durations may be used as well, e.g., 10 seconds.

The pre-processing can comprise determining for each of the audio segments whether to include it in the cough audio data.

Determining for each of the audio segments whether to include it in the cough audio data can comprise including in the cough audio data one or more of the audio segments for which the cough detector determines a positive indication on whether the audio segment contains a cough sound. Simply put, for each audio segment, if a cough sound may be detected therein, it may be included in the cough audio data.

Determining for each audio segment whether to include it in the cough audio data can comprise filtering out one or more of the audio segments for which the cough detector determines a positive indication on whether the audio segment contains a non-cough sound. Simply put, for each audio segment, if a non-cough sound may be detected therein, it may be disregarded, i.e., not included in the cough audio data.

The pre-processing can comprise determining, for each of the audio segments, a respective cough indicator indicative of whether the respective audio segment contains a cough sound and wherein determining for each of the audio segments whether to include it in the cough audio data may be based on the respective cough indicator. The cough indicator of each audio segment may, for example, be a likelihood of the presence of a cough sound in the respective audio segment.

The cough audio data can comprise for each audio segment included therein the respective cough indicator. This can facilitate further processing of the cough audio data.

The pre-processing can comprise determining, for each of the audio segments, a non-cough indicator indicative of whether the respective audio segment contains a non-cough sound and wherein determining for each of the audio segments whether to include it in the cough audio data may be based on the non-cough indicator. The non-cough indicator of each audio segment may, for example, be a likelihood of the presence of a non-cough sound in the respective audio segment.

The cough audio data can comprise for each audio segment included therein the respective non-cough indicator. This can facilitate further processing of the cough audio data.

The cough detector can comprise multiple stage detectors arranged sequentially such that each subsequent stage detector receives as input the output of a preceding stage detector. In other words, the detecting of cough sounds and/or the filtering out of non-cough sounds may be performed in multiple sequential stages. This may allow defining coarser (and thus less computationally complex) filters in initial stages and sequentially making these filters less coarse (and thus more computationally complex). This may be particularly advantageous as it may allow the method to be carried out locally at the vicinity of the user, wherein the computational resources may be more limited, as well as, remotely in a cloud server wherein more computational resources may be available.

Additionally, having multiple stage detectors allows sensitive information (e.g., speech) to be directly filtered out locally and the rest of the filtering can be carried out remotely. This way, privacy of the targeted user is maintained, while at the same time a more efficient cough detection is performed remotely.

Each stage detector may comprise a (respective) processing unit which may be singular or plural, and may be, but not limited to, a CPU (central processing unit), GPU (graphical processing unit), DSP (digital signal processor), APU (accelerator processing unit), ASIC (application-specific integrated circuit), ASIP (application-specific instruction-set processor) or FPGA (field programable gate array). The processing unit may comprise one or more micro-controller units.

Further, each stage detector may comprise a (respective) memory component which may be singular or plural, and may be, but is not limited to, a volatile or non-volatile memory, such as a random-access memory (RAM), Dynamic RAM (DRAM), Synchronous Dynamic RAM (SDRAM), static RAM (SRAM), Flash Memory, Magneto-resistive RAM (MRAM), Ferroelectric RAM (F-RAM), or Parameter RAM (P-RAM).

Each stage detector may further comprise (respective) input and output interfaces for exchanging data electronically.

Each subsequent stage detector may be more complex than the preceding one.

One of the stage detectors may be a first stage detector without any preceding stage detector and wherein one of the stage detectors may be a final stage detector without any subsequent stage detector and wherein the first stage detector receives as input the source audio data and wherein the final stage detector outputs the cough audio data. That is, the source audio data may represent unfiltered audio data, whereas the cough audio data may represent completely filtered audio data. Ideally, the cough audio data may only consist of cough sounds. It will be understood that in practice this may not be the case due to imperfections and/or tolerances of the algorithms used to detect cough sounds and/or to filter non-cough sounds. In between the source audio data and the cough audio data there may be one or more versions of the audio data, wherein each subsequent version may likely comprise fewer cough sounds then the preceding one.

Each stage detector may determine for each audio segment that it receives whether to include it in the cough audio data.

It will be understood that each stage detector can respectively make said determination as discussed above with reference to the cough detector in general.

The pre-processing can comprise generating potentially cough audio data based on the source audio data and generating the cough audio data based on the potentially cough data. In other words, two stage detectors may be used, one receiving source audio data and generating potentially cough audio data and the other receiving the potentially cough audio data and generating cough audio data.

The potentially cough audio data may interchangeably be referred to as potentially cough data.

The potentially cough data may contain less non-cough sounds than the source audio data. It will be understood that the potentially cough data may, on average, contain less non-cough sounds than the source audio data. That is, the potentially cough audio data can be more likely to contain less non-cough sounds than the source audio data.

The cough audio data contains less non-cough sounds than the potentially cough data. It will be understood that the cough audio data may, on average, contain less non-cough sounds than the potentially cough data. That is, the cough audio data can be more likely to contain less non-cough sounds than the potentially cough audio data.

The cough detector can comprise an edge detector and wherein generating the potentially cough audio data based on the source audio data may be performed by the edge detector. Using an edge detector can be advantageous as it can be more easily provided at the vicinity of the targeted user. Furthermore, the edge detector facilitates filtering out non-cough sounds directly at edge devices being in the vicinity of the targeted user and/or under ownership and control of the targeted user. Thus, maintaining privacy of the targeted user can be facilitated.

The terms edge detector and edge cough detector are used interchangeably.

The edge detector may comprise a processing unit which may be singular or plural, and may be, but not limited to, a CPU (central processing unit), GPU (graphical processing unit), DSP (digital signal processor), APU (accelerator processing unit), ASIC (application-specific integrated circuit), ASIP (application-specific instruction-set processor) or FPGA (field programable gate array). The processing unit may comprise one or more micro-controller units.

Further, the edge detector may comprise a memory component which may be singular or plural, and may be, but is not limited to, a volatile or non-volatile memory, such as a random-access memory (RAM), Dynamic RAM (DRAM), Synchronous Dynamic RAM (SDRAM), static RAM (SRAM), Flash Memory, Magneto-resistive RAM (MRAM), Ferroelectric RAM (F-RAM), or Parameter RAM (P-RAM).

The edge detector may further comprise input and output interfaces for exchanging data electronically.

The edge detector may be the first stage detector. That is, if multiple stage detectors are used, the edge detector may be the entry point into the infrastructure of the multiple stage detectors.

The pre-processing can comprise partitioning, with the edge detector, the source audio data in the audio segments. That is, it can be the edge detector that partitions the source audio data in the audio segments.

The pre-processing can comprise determining, with the edge detector, for each of the audio segments in the source audio data whether to include it in the potentially cough audio data.

Determining, with the edge detector, for each of the audio segments in the source audio data whether to include it in the potentially cough audio data can comprise including in the potentially cough audio data one or more of the audio segments in the source audio data for which the edge detector determines a positive indication on whether the audio segment contains a cough sound.

Determining, with the edge detector, for each of the audio segments in the source audio data whether to include it in the potentially cough audio data can comprise filtering out one or more of the audio segments in the source audio data for which the edge detector determines a positive indication on whether the audio segment contains a non-cough sound.

In particular, the edge detector can be configured to filter out speech sounds.

The pre-processing can comprise determining, with the edge detector, for each of the audio segments in the source audio data, a respective edge cough indicator indicative of whether the respective audio segment contains a cough sound and wherein determining for each of the audio segments in the source audio data whether to include it in the potentially cough audio data may be based on the respective edge cough indicator.

The potentially cough audio data can comprise for each audio segment included therein the respective edge cough indicator. This may facilitate the operation of a subsequent stage detector, e.g., the operation of the cloud cough detector.

The pre-processing can comprise determining, with the edge detector, for each of the audio segments in the source audio data, an edge non-cough indicator indicative of whether the respective audio segment contains a non-cough sound and wherein determining for each of the audio segments in the source audio data whether to include it in the potentially cough audio data may be based on the edge non-cough indicator.

The potentially cough audio data can comprise for each audio segment included therein the respective edge non-cough indicator. This may facilitate the operation of a subsequent stage detector, e.g., the operation of the cloud cough detector.

The potentially cough audio data further can comprise for each audio segment included therein respective segment metadata. This may facilitate the operation of a subsequent stage detector, e.g., the operation of the cloud cough detector.

The segment metadata can comprise a recorded time of the respective audio segment.

The segment metadata can comprise a user unique ID of the targeted user.

The pre-processing can comprise generating, with the edge detector, for each audio segment, a respective edge audio feature characterizing the audio segment and wherein determining for each of the audio segments in the source audio data whether to include it in the potentially cough audio data may be based on the respective edge audio feature.

The edge audio feature may be indicative of a spectrogram of the respective audio segment, wherein the spectrogram may be preferably a Mel spectrogram, more preferably a log-Mel-spectrogram, even more preferably an intensity normalized log-Mel spectrogram.

The edge audio feature may be generated by converting the spectrogram of the respective audio segment into a two-dimensional spectrogram image, preferably a single-channel spectrogram image.

Determining, with the edge detector, for each of the audio segments in the source audio data whether to include it in the potentially cough audio data can comprise utilizing an edge machine learning architecture, preferably based on a convolutional neural network.

The edge machine learning architecture may be configured to be executed in resource constraint devices. This can be particularly advantageous because computational resources at edge devices can be limited. Therefore, the edge machine learning architecture being configured for resource constraint devices may be efficiently executed by edge devices.

The edge machine learning architecture may be configured to detect a visual feature in a two-dimensional image.

Determining for each of the audio segments in the source audio data whether to include it in the potentially cough audio data based on the respective edge audio feature may be performed by utilizing the edge machine learning architecture.

The method can comprise generating, with the edge machine learning architecture the edge cough indicator and/or the edge non-cough indicator.

The edge machine learning architecture may be trained using an edge training dataset, wherein the edge training dataset may be generated based on a labelled audio dataset comprising cough and non-cough sounds.

The method can comprise relabeling at least a part of the labelled audio data set to increase labelling accuracy and/or eliminate pauses within the labelled audio dataset with a duration longer than a pause threshold. This can advantageously increase the quality of the dataset which in turn may improve the cough detection performance.

The method can comprise generating the edge training dataset further based on augmented training data, wherein the augmented training data may be generated by applying one or more data augmentation techniques to the labelled audio dataset, such as, audio speed variation, audio pitch variation, noise augmentation, room impulse response, audio gain variation, time masking and/or frequency masking.

The edge training dataset may be configured such that the edge machine learning architecture prevents sensitive sound like speech, cry, and so on being detected as cough. Hence, sensitive sounds are not transmitted to cloud or other downstream tasks. This may be achieved via labeling, relabeling and/or augmenting the training dataset, as discussed.

The cough detector can comprise a cloud detector and wherein generating the cough audio data based on the potentially cough audio data may be performed by the cloud detector. Using a cloud detector can be advantageous as it can provide large computational resources. Therefore, more complex cough detecting algorithms may be used.

The terms cloud detector and cloud cough detector are used interchangeably.

The cloud detector may comprise a processing unit which may be singular or plural, and may be, but not limited to, a CPU (central processing unit), GPU (graphical processing unit), DSP (digital signal processor), APU (accelerator processing unit), ASIC (application-specific integrated circuit), ASIP (application-specific instruction-set processor) or FPGA (field programable gate array). The processing unit may comprise one or more micro-controller units.

Further, the cloud detector may comprise a memory component which may be singular or plural, and may be, but is not limited to, a volatile or non-volatile memory, such as a random-access memory (RAM), Dynamic RAM (DRAM), Synchronous Dynamic RAM (SDRAM), static RAM (SRAM), Flash Memory, Magneto-resistive RAM (MRAM), Ferroelectric RAM (F-RAM), or Parameter RAM (P-RAM).

The cloud detector may further comprise input and output interfaces for exchanging data electronically.

The cloud detector may be the final stage detector.

The cloud detector can comprise more computational resources than the edge detector.

The pre-processing can comprise determining, with the cloud detector, for each of the audio segments in the potentially cough audio data whether to include it in the cough audio data.

Determining, with the cloud detector, for each of the audio segments in the potentially cough audio data whether to include it in the cough audio data can comprise including in the cough audio data one or more of the audio segments in the potentially cough audio data for which the cloud detector determines a positive indication on whether the audio segment contains a cough sound.

Determining, with the cloud detector, for each of the audio segments in the potentially cough audio data whether to include it in the cough audio data can comprise filtering out one or more of the audio segments in the potentially cough audio data for which the cloud detector determines a positive indication on whether the audio segment contains a non-cough sound.

The pre-processing can comprise determining, with the cloud detector, for each of the audio segments in the potentially cough audio data, a respective cloud cough indicator indicative of whether the respective audio segment contains a cough sound and wherein determining for each of the audio segments in the potentially cough audio data whether to include it in the cough audio data may be based on the respective cloud cough indicator.

The cough audio data can comprise for each audio segment included therein the respective cloud cough indicator. This may facilitate subsequent tasks, e.g., targeted user cough detection.

The pre-processing can comprise determining, with the cloud detector, for each of the audio segments in the potentially cough audio data, a cloud non-cough indicator indicative of whether the respective audio segment contains a non-cough sound and wherein determining for each of the audio segments in the potentially cough audio data whether to include it in the cough audio data may be based on the cloud non-cough indicator.

The cough audio data can comprise for each audio segment included therein the respective cloud non-cough indicator. This may facilitate subsequent tasks, e.g., targeted user cough detection.

The cough audio data further can comprise for each audio segment included therein respective segment metadata. This may facilitate subsequent tasks, e.g., targeted user cough detection.

The segment metadata can comprise a recorded time of the respective audio segment.

The segment metadata can comprise a user unique ID of the targeted user.

The pre-processing can comprise generating, with the cloud detector, for each audio segment, a respective cloud audio feature characterizing the audio segment and wherein determining for each of the audio segments in the potentially cough audio data whether to include it in the cough audio data may be based on the respective cloud audio feature.

The cloud audio feature may be indicative of a spectrogram of the respective audio segment, wherein the spectrogram may be preferably a Mel spectrogram, more preferably a log-Mel-spectrogram, even more preferably an intensity normalized log-Mel spectrogram.

Determining, with the cloud detector, for each of the audio segments in the potentially cough audio data whether to include it in the cough audio data can comprise utilizing a cloud machine learning architecture, preferably based on Transformer models.

The cloud machine learning architecture may be configured to be executed in resource extensive devices. This may provide more accurate cough detection and/or more accurate filtering of non-cough sounds.

Determining for each of the audio segments in the potentially cough audio data whether to include it in the cough audio data based on the respective cloud audio feature may be performed by utilizing the cloud machine learning architecture.

The method can comprise generating, with the cloud machine learning architecture the cloud cough indicator and/or the cloud non-cough indicator.

The cloud machine learning architecture may be trained using a cloud training dataset, wherein the cloud training dataset may be generated based on a labelled audio dataset comprising cough and non-cough sounds.

The method can comprise relabeling at least a part of the labelled audio data set to increase labelling accuracy and/or eliminate pauses within the labelled audio dataset with a duration longer than a pause threshold.

The method can comprise generating the cloud training dataset further based on augmented training data, wherein the augmented training data may be generated by applying one or more data augmentation techniques to the labelled audio dataset, such as, audio speed variation, audio pitch variation, noise augmentation, room impulse response, audio gain variation, time masking and/or frequency masking.

The cloud training dataset may be configured such that the cloud machine learning architecture prevents sensitive sound like speech, cry, and so on being detected as cough. Hence, sensitive sounds are not to other downstream tasks (e.g., targeted user cough detection). This may be achieved via labeling, relabeling and/or augmenting the training dataset, as discussed.

The edge training dataset and the cloud training dataset may be the same. It will be understood that this is not a necessary requirement and that the edge training dataset may be different from the cloud training dataset.

The edge detector may be part of an edge system. The edge system may comprise one or more devices positioned in the vicinity of the targeted user. It will be understood that this may include devices positioned on the targeted user, such as, wearable devices.

The edge system can comprise the audio recorder.

The edge system can comprise an edge communication device.

The method can comprise transmitting with the edge communication device the potentially cough audio data to the cloud detector. Standardized communication protocols may be used herein.

The target cough detector may be part of a cloud system. The cloud system may comprise one or more devices remotely positioned with respect to the targeted user. The cloud system may be configured to perform cloud computing. The cloud system may comprise one or more servers.

The cloud system can comprise the targeted cough detector.

The method can comprise transmitting the cough audio data from the cloud detector to the targeted cough detector. Standardized communication protocols may be used herein.

The processing can comprise differentiating cough of the targeted user from cough of other individuals. That is, the processing may comprise isolating the cough of the targeted user from other cough sounds.

The cough audio clip can comprise one or more of the audio segments. The cough audio clip may be generated based on one or more audio segments. The cough audio clip may also comprise a portion of an audio segment.

The cough audio clip can comprise one or more of the audio segments included in the cough audio data. The cough audio clip may be generated based on one or more audio segments included in the cough audio data. The cough audio clip may also comprise a portion of an audio segment included in the cough audio data.

The processing can comprise computing a comparison parameter corresponding to the cough audio clip, comparing the comparison parameter with a reference comparison parameter corresponding to the targeted user, and generating the result based on the comparison. The reference comparison parameter may be configured such that it uniquely corresponds to the targeted user. The reference comparison parameter may represent characteristics or features of the cough of the targeted user. Thus, comparison with the reference comparison parameter may allow determining whether the cough audio clip contain cough of the targeted user.

The comparison parameter and the reference comparison parameter can comprise the same structure. This can facilitate the comparison between the two parameters. For example, the comparison parameter and the reference comparison parameter may be descriptive of same features.

The comparison parameter may be a vector representation, such as, an embedding, of the cough audio clip.

The method can comprise computing a similarity score between the comparison parameter and the reference comparison parameter.

Generating the result can comprise generating a positive indication that the cough audio clip contains a cough sound of the targeted user if the similarity score may be higher than a similarity threshold and generating a negative indication otherwise.

The method can comprise initializing the reference comparison parameter and wherein said initializing can comprise generating the reference comparison parameter based on enrolment cough data comprising cough recordings of the targeted user. The enrolment cough data may comprise audio clips for which it is known that they comprise cough of the targeted user. For example, the enrolment cough data may comprise recordings by the targeted user while coughing. The reference comparison parameter, thus initialized, may provide a first estimation on the characteristics of the cough of the targeted user.

The method can comprise obtaining the enrolment cough data while the targeted user may be coughing. This can ensure that the enrolment cough data comprise cough of the targeted user. This can be advantageous because at this initial stage, wherein the reference comparison parameter of the targeted user may not yet be known, it may not be possible to automatically detect cough of the targeted user. In fact, the enrolment cough data may allow for the reference comparison parameter to be computed and may thereafter allow automatic detection of cough of the targeted user.

The enrolment cough data can comprise a plurality of enrolment cough audio clips and wherein generating the reference comparison parameter based on enrolment cough data can comprise computing the reference comparison parameter based on enrolment comparison parameters respectively corresponding to the enrolment cough audio clips. The initialized reference comparison parameter may thus be more accurate, i.e., may better represent characteristics of the cough of the targeted user, as, for example, when computed based on a single enrolment cough audio clip.

It will be understood that the enrolment cough audio clips can comprise a similar data structure to the cough audio clip. Thus, the same algorithm as for computing the comparison parameter of the cough audio clip can be used to compute reference comparison parameters of the enrolment cough audio clips.

The method can comprise updating the reference comparison parameter, preferably routinely. This can allow adapting the reference comparison parameter particularly in view of changes that may happen to cough of the targeted user over time. Thus, as cough of the targeted user changes, the reference comparison parameter can be updated accordingly.

Updating the reference comparison parameter may be based on past cough audio clips, wherein the past cough audio clips may be generated prior to the cough audio clip. In other words, the reference comparison parameter may be dynamically updated.

It will be understood that the past cough audio clips can comprise a similar data structure to the cough audio clip. In fact, the past cough audio clips may be cough audio clips generated in the past.

The past cough audio clips can comprise one or more of the audio segments included in the cough audio data.

Each of the past cough audio clips used for updating the reference comparison parameter can comprise a respective comparison parameter, the similarity score of which may be higher than an updating threshold. In other words, only past cough audio clips for which a high similarity score is determined may be used to update the reference comparison parameter. On the one hand this makes it more likely that clips containing cough of the targeted user are used to update the reference comparison parameter. Additionally, outliers may be disregarded and not used for the update. Outliers may be clips containing cough which although may belong to the targeted user, temporally and substantially deviate from the targeted user's normal cough.

Updating the reference comparison parameter may be triggered after the past cough audio clips reach a predetermined number.

Updating the reference comparison parameter based on past cough audio clips can comprise updating the reference comparison parameter based on comparison parameters respectively corresponding to the past cough audio clips.

The targeted cough detector utilizes a machine learning algorithm trained on cough data. The cough data used for training the machine learning algorithm of the targeted cough detector may comprise a plurality of cough sounds of multiple individuals.

The machine learning algorithm utilized by the targeted cough detector can comprise a speaker verification algorithm.

The speaker verification algorithm may be pre-trained using speech audio data to verify speaker. That is the speaker verification algorithm may be configured to determine whether the speech audio data contain speech of a targeted speaker.

Speech audio data may refer to audio data containing speech sound.

The speaker verification algorithm can further be trained using cough sounds. For example, a cough dataset may be used to train the speaker verification algorithm. Thus, the speaker verification algorithm may be fine-tuned for cough recognition of the targeted user.

The method can comprise configuring the speaker verification algorithm to recognize cough of the targeted user.

Configuring the speaker verification algorithm to recognize cough of the targeted user can comprise the initializing of the reference comparison parameter and the updating of the reference comparison parameter using past cough audio data. Past cough audio data may refer to cough audio data obtained prior to the cough audio clip.

The method can comprise generating cough metrics based on audio data containing cough of the targeted user.

The cough metrics may be indicative of the coughing behavior of the targeted user.

The cough metrics may be indicative of a cough pattern and/or of a cough frequency.

The cough metrics may be indicative of a cough-related disease, such as, a respiratory disease.

The method can comprise accumulating the cough metric over time.

The method may be a computer implemented method.

The present invention further relates to a system for detecting cough of a targeted user. The system comprises a targeted cough detector configured to process a cough audio clip and to generate based thereon a result indicative of whether the cough audio clip contains a cough sound of the targeted user.

The system comprises corresponding features to the method as discussed above and below. For the sake of brevity, a detailed discussion of these features is omitted herein.

The system can be configured to carry out the method of the present invention.

Similarly, the method can be carried out by the system of the present invention.

The present invention further relates to a computer program product comprising instructions which, when the program is executed by the system according to the present invention, cause the system to carry out the method according to the present invention.

The present invention further relates to a computer-readable storage medium comprising instructions which, when executed by the system according to the present invention, cause the system to carry out the method according to the present invention.

The present invention is also defined by the following numbered embodiments.

Below, method embodiments will be discussed. These embodiments are abbreviated by the letter "M" followed by a number. When reference is herein made to method embodiments, these embodiments are meant.

M1. A method to detect cough of a targeted user, the method comprising:

processing a cough audio clip with a targeted cough detector and
generating based thereon a result indicative of whether the cough audio clip contains a cough sound of the targeted user.

M2. The method according to any of the preceding embodiments, wherein the method comprises obtaining source audio data, wherein the source audio data comprise the cough audio clip.

M3. The method according to the preceding embodiment, wherein obtaining the source data comprises generating the source audio data with an audio recorder.

M4. The method according to the preceding embodiment, wherein the method comprises the audio recorder continuously recording the source audio data.

M5. The method according to any of the 2 preceding embodiments, wherein the audio recorder is a portable device, a wearable device or a stationary device.

M6. The method according to any of the 3 preceding embodiments, wherein the audio recorder comprises at least one auxiliary sensor configured to sense auxiliary data about a surrounding of the at least one auxiliary sensor.

M7. The method according to the preceding embodiment wherein the auxiliary data are indicative of a humidity, pollution and/or presence of allergens in the surrounding of the at least one auxiliary sensor.

M8. The method according to any of the preceding embodiments and with the features of embodiment M2, wherein the method comprises pre-processing the source audio data with a cough detector to obtain cough audio data, wherein the cough audio data comprise the cough audio clip.

M9. The method according to the preceding embodiment, wherein the pre-processing is performed such that the cough audio data contain less non-cough sounds than the source audio data.

M10. The method according to any of the 2 preceding embodiments, wherein the cough detector is configured to detect cough sounds.

M11. The method according to any of the 3 preceding embodiments, wherein the cough detector is configured to detect non-cough sounds,

preferably a plurality of non-cough sound types,
more preferably non-cough sound types, wherein each corresponds to a respective sound producible by a human.

M12. The method according to any of the preceding embodiments and with the features of embodiment M2, wherein the source audio data is partitioned in audio segments.

M13. The method according to the preceding embodiment, wherein the audio segments overlap.

M14. The method according to any of the 2 preceding embodiments, wherein the audio segments comprise a constant duration, preferably at least 0.5 seconds and at most 5 seconds, more preferably at least 1 second and at most 2 seconds.

M15. The method according to any of the 3 preceding embodiments and with the features of embodiment M8, wherein the pre-processing comprises determining for each of the audio segments whether to include it in the cough audio data.

M16. The method according to the preceding embodiment, wherein determining for each of the audio segments whether to include it in the cough audio data comprises

including in the cough audio data one or more of the audio segments for which the cough detector determines a positive indication on whether the audio segment contains a cough sound.

M17. The method according to any of the 2 preceding embodiments, wherein determining for each audio segment whether to include it in the cough audio data comprises

filtering out one or more of the audio segments for which the cough detector determines a positive indication on whether the audio segment contains a non-cough sound.

M18. The method according to any of the 3 preceding embodiments, wherein the preprocessing comprises determining, for each of the audio segments, a respective cough indicator indicative of whether the respective audio segment contains a cough sound and

wherein determining for each of the audio segments whether to include it in the cough audio data is based on the respective cough indicator.

M19. The method according to the preceding embodiment, wherein the cough audio data comprise for each audio segment included therein the respective cough indicator.

M20. The method according to any of the 5 preceding embodiments, wherein the preprocessing comprises determining, for each of the audio segments, a non-cough indicator indicative of whether the respective audio segment contains a non-cough sound and

wherein determining for each of the audio segments whether to include it in the cough audio data is based on the non-cough indicator.

M21. The method according to the preceding embodiment, wherein the cough audio data comprise for each audio segment included therein the respective non-cough indicator.

M22. The method according to any of the preceding embodiments and with the features of embodiment M8, wherein the cough detector comprises multiple stage detectors arranged sequentially such that each subsequent stage detector receives as input the output of a preceding stage detector.

M23. The method according to the preceding embodiment, wherein each subsequent stage detector is more complex than the preceding one.

M24. The method according to any of the 2 preceding embodiments, wherein one of the stage detectors is a first stage detector without any preceding stage detector and wherein one of the stage detectors is a final stage detector without any subsequent stage detector and wherein the first stage detector receives as input the source audio data and wherein the final stage detector outputs the cough audio data.

M25. The method according to any of the 3 preceding embodiments and with the features of embodiment M15, wherein each stage detector determines for each audio segment that it receives whether to include it in the cough audio data.

It will be understood that each stage detector can respectively make said determination as discussed in embodiments M16 to M21.

M26. The method according to any of the preceding embodiments and with the features of embodiment M8, wherein the pre-processing comprises

generating potentially cough audio data based on the source audio data and
generating the cough audio data based on the potentially cough data.

M27. The method according to the preceding embodiment, wherein the potentially cough data contains less non-cough sounds than the source audio data.

M28. The method according to any of the 2 preceding embodiments, wherein the cough audio data contains less non-cough sounds than the potentially cough data.

M29. The method according to any of the preceding embodiments and with the features of embodiment M8 and M26, wherein the cough detector comprises an edge detector and wherein generating the potentially cough audio data based on the source audio data is performed by the edge detector.

M30. The method according to the preceding embodiment and with the features of embodiment M24, wherein the edge detector is the first stage detector.

M31. The method according to any of the 2 preceding embodiments and with the features of embodiment M12, wherein the pre-processing comprises partitioning, with the edge detector, the source audio data in the audio segments.

M32. The method according to any of the 3 preceding embodiments and with the features of embodiment M12, wherein the pre-processing comprises determining, with the edge detector, for each of the audio segments in the source audio data whether to include it in the potentially cough audio data.

M33. The method according to the preceding embodiment, wherein determining, with the edge detector, for each of the audio segments in the source audio data whether to include it in the potentially cough audio data comprises including in the potentially cough audio data one or more of the audio segments in the source audio data for which the edge detector determines a positive indication on whether the audio segment contains a cough sound.

M34. The method according to any of the 2 preceding embodiments, wherein determining, with the edge detector, for each of the audio segments in the source audio data whether to include it in the potentially cough audio data comprises filtering out one or more of the audio segments in the source audio data for which the edge detector determines a positive indication on whether the audio segment contains a non-cough sound.

In particular, the edge detector can be configured to filter out speech sounds.

M35. The method according to any of the 3 preceding embodiments, wherein the preprocessing comprises determining, with the edge detector, for each of the audio segments in the source audio data, a respective edge cough indicator indicative of whether the respective audio segment contains a cough sound and wherein determining for each of the audio segments in the source audio data whether to include it in the potentially cough audio data is based on the respective edge cough indicator.

M36. The method according to the preceding embodiment, wherein the potentially cough audio data comprise for each audio segment included therein the respective edge cough indicator.

M37. The method according to any of the 5 preceding embodiments, wherein the preprocessing comprises determining, with the edge detector, for each of the audio segments in the source audio data, an edge non-cough indicator indicative of whether the respective audio segment contains a non-cough sound and wherein determining for each of the audio segments in the source audio data whether to include it in the potentially cough audio data is based on the edge non-cough indicator.

M38. The method according to the preceding embodiment, wherein the potentially cough audio data comprises for each audio segment included therein the respective edge non-cough indicator.

M39. The method according to any of the 7 preceding embodiments, wherein the potentially cough audio data further comprises for each audio segment included therein respective segment metadata.

M40. The method according to the preceding embodiment, wherein the segment metadata comprises a recorded time of the respective audio segment.

M41. The method according to any of the 2 preceding embodiments, wherein the segment metadata comprise a user unique ID of the targeted user.

M42. The method according to any of the preceding embodiments and with the features of embodiment M32, wherein the pre-processing comprises

generating, with the edge detector, for each audio segment, a respective edge audio feature characterizing the audio segment and
wherein determining for each of the audio segments in the source audio data whether to include it in the potentially cough audio data is based on the respective edge audio feature.

M43. The method according to the preceding embodiment, wherein the edge audio feature is indicative of a spectrogram of the respective audio segment, wherein the spectrogram is preferably a Mel spectrogram, more preferably a log-Mel-spectrogram, even more preferably an intensity normalized log-Mel spectrogram.

M44. The method according to the preceding embodiment, wherein the edge audio feature is generated by converting the spectrogram of the respective audio segment into a two-dimensional spectrogram image, preferably a single-channel spectrogram image.

M45. The method according to any of the preceding embodiments and with the features of embodiment M32, wherein determining, with the edge detector, for each of the audio segments in the source audio data whether to include it in the potentially cough audio data comprises utilizing an edge machine learning architecture, preferably based on a convolutional neural network.

M46. The method according to the preceding embodiment, wherein the edge machine learning architecture is configured to be executed in resource constraint devices.

M47. The method according to any of the 2 preceding embodiments, wherein the edge machine learning architecture is configured to detect a visual feature in a two-dimensional image.

M48. The method according to any of the 3 preceding embodiments and with the features of embodiment M42, wherein determining for each of the audio segments in the source audio data whether to include it in the potentially cough audio data based on the respective edge audio feature is performed by utilizing the edge machine learning architecture.

M49. The method according to any of the 4 preceding embodiments and with the features of embodiments M35 and/or M37, wherein the method comprises generating, with the edge machine learning architecture the edge cough indicator and/or the edge non-cough indicator.

M50. The method according to any of the 5 preceding embodiments, wherein the edge machine learning architecture is trained using an edge training dataset, wherein the edge training dataset is generated based on a labelled audio dataset comprising cough and non-cough sounds.

M51. The method according to the preceding embodiment, wherein the method comprises relabeling at least a part of the labelled audio data set to increase labelling accuracy and/or eliminate pauses within the labelled audio dataset with a duration longer than a pause threshold.

M52. The method according to any of the 2 preceding embodiments, wherein the method comprises generating the edge training dataset further based on augmented training data, wherein the augmented training data are generated by applying one or more data augmentation techniques to the labelled audio dataset, such as, audio speed variation, audio pitch variation, noise augmentation, room impulse response, audio gain variation, time masking and/or frequency masking.

M53. The method according to any of the preceding embodiments and with the features of embodiment M8 and M26, wherein the cough detector comprises a cloud detector and wherein generating the cough audio data based on the potentially cough audio data is performed by the cloud detector.

M54. The method according to the preceding embodiment and with the features of embodiment M24, wherein the cloud detector is the final stage detector.

M55. The method according to any of the 2 preceding embodiments and with the features of embodiment M29, wherein the cloud detector comprises more computational resources than the edge detector.

M56. The method according to any of the 3 preceding embodiments and with the features of embodiment M12, wherein the pre-processing comprises determining, with the cloud detector, for each of the audio segments in the potentially cough audio data whether to include it in the cough audio data.

M57. The method according to the preceding embodiment, wherein determining, with the cloud detector, for each of the audio segments in the potentially cough audio data whether to include it in the cough audio data comprises including in the cough audio data one or more of the audio segments in the potentially cough audio data for which the cloud detector determines a positive indication on whether the audio segment contains a cough sound.

M58. The method according to any of the 2 preceding embodiments, wherein determining, with the cloud detector, for each of the audio segments in the potentially cough audio data whether to include it in the cough audio data comprises

filtering out one or more of the audio segments in the potentially cough audio data for which the cloud detector determines a positive indication on whether the audio segment contains a non-cough sound.

M59. The method according to any of the 3 preceding embodiments, wherein the preprocessing comprises determining, with the cloud detector, for each of the audio segments in the potentially cough audio data, a respective cloud cough indicator indicative of whether the respective audio segment contains a cough sound and wherein determining for each of the audio segments in the potentially cough audio data whether to include it in the cough audio data is based on the respective cloud cough indicator.

M60. The method according to the preceding embodiment, wherein the cough audio data comprises for each audio segment included therein the respective cloud cough indicator.

M61. The method according to any of the 5 preceding embodiments, wherein the preprocessing comprises determining, with the cloud detector, for each of the audio segments in the potentially cough audio data, a cloud non-cough indicator indicative of whether the respective audio segment contains a non-cough sound and wherein determining for each of the audio segments in the potentially cough audio data whether to include it in the cough audio data is based on the cloud non-cough indicator.

M62. The method according to the preceding embodiment, wherein the cough audio data comprises for each audio segment included therein the respective cloud non-cough indicator.

M63. The method according to any of the 7 preceding embodiments, wherein the cough audio data further comprises for each audio segment included therein respective segment metadata.

M64. The method according to the preceding embodiment, wherein the segment metadata comprise a recorded time of the respective audio segment.

M65. The method according to any of the 2 preceding embodiments, wherein the segment metadata comprise a user unique ID of the targeted user.

M66. The method according to any of the preceding embodiments and with the features of embodiment M56, wherein the pre-processing comprises

generating, with the cloud detector, for each audio segment, a respective cloud audio feature characterizing the audio segment and

wherein determining for each of the audio segments in the potentially cough audio data whether to include it in the cough audio data is based on the respective cloud audio feature.

M67. The method according to the preceding embodiment, wherein the cloud audio feature is indicative of a spectrogram of the respective audio segment, wherein the spectrogram is preferably a Mel spectrogram, more preferably a log-Mel-spectrogram, even more preferably an intensity normalized log-Mel spectrogram.

M68. The method according to any of the preceding embodiments and with the features of embodiment M56, wherein determining, with the cloud detector, for each of the audio segments in the potentially cough audio data whether to include it in the cough audio data comprises utilizing a cloud machine learning architecture, preferably based on Transformer models.

M69. The method according to the preceding embodiment, wherein the cloud machine learning architecture is configured to be executed in resource extensive devices.

M70. The method according to any of the 2 preceding embodiments and with the features of embodiment M66, wherein determining for each of the audio segments in the potentially cough audio data whether to include it in the cough audio data based on the respective cloud audio feature is performed by utilizing the cloud machine learning architecture.

M71. The method according to any of the 4 preceding embodiments and with the features of embodiments M59 and/or M61, wherein the method comprises generating, with the cloud machine learning architecture the cloud cough indicator and/or the cloud non-cough indicator.

M72. The method according to any of the 5 preceding embodiments, wherein the cloud machine learning architecture is trained using a cloud training dataset, wherein the cloud training dataset is generated based on a labelled audio dataset comprising cough and non-cough sounds.

M73. The method according to the preceding embodiment, wherein the method comprises relabeling at least a part of the labelled audio data set to increase labelling accuracy and/or eliminate pauses within the labelled audio dataset with a duration longer than a pause threshold.

M74. The method according to any of the 2 preceding embodiments, wherein the method comprises generating the cloud training dataset further based on augmented training data, wherein the augmented training data are generated by applying one or more data augmentation techniques to the labelled audio dataset, such as, audio speed variation, audio pitch variation, noise augmentation, room impulse response, audio gain variation, time masking and/or frequency masking.

M75. The method according to any of the 3 preceding embodiments and with the features of embodiment M50, wherein the edge training dataset and the cloud training dataset are the same.

However, this is not a requirement and the edge training dataset and the cloud training dataset may be different.

M76. The method according to any of the preceding embodiments and with the features of embodiment M29, wherein the edge detector is part of an edge system.

M77. The method according to the preceding embodiment and with the features of embodiment M3, wherein the edge system comprises the audio recorder.

M78. The method according to any of the 2 preceding embodiments, wherein the edge system comprises an edge communication device.

M79. The method according to the preceding embodiment and with the features of embodiment M53, wherein the method comprises transmitting with the edge communication device the potentially cough audio data to the cloud detector.

M80. The method according to any of the preceding embodiments, wherein the target cough detector is part of a cloud system.

M81. The method according to the preceding embodiment and with the features of embodiment M53, wherein the cloud system comprises the targeted cough detector.

M82. The method according to any of the preceding embodiments and with the features of embodiment M53, wherein the method comprises transmitting the cough audio data from the cloud detector to the targeted cough detector.

M83. The method according to any of the preceding embodiments, wherein the processing comprises differentiating cough of the targeted user from cough of other individuals.

M84. The method according to any of the preceding embodiments and with the features of embodiment M12, wherein the cough audio clip comprises one or more of the audio segments.

M85. The method according to any of the preceding embodiments and with the features of embodiment M15, wherein the cough audio clip comprises one or more of the audio segments included in the cough audio data.

M86. The method according to any of the preceding embodiments, wherein the processing comprises

computing a comparison parameter corresponding to the cough audio clip,
comparing the comparison parameter with a reference comparison parameter corresponding to the targeted user,
generating the result based on the comparison.

M87. The method according to the preceding embodiment, wherein the comparison parameter and the reference comparison parameter comprise the same structure.

M88. The method according to any of the 2 preceding embodiments, wherein the comparison parameter is a vector representation, such as, an embedding, of the cough audio clip.

M89. The method according to any of the 3 preceding embodiments, wherein the method comprises computing a similarity score between the comparison parameter and the reference comparison parameter.

M90. The method according to the preceding embodiment, wherein generating the result comprises

generating a positive indication that the cough audio clip contains a cough sound of the targeted user if the similarity score is higher than a similarity threshold and
generating a negative indication otherwise.

M91. The method according to any of the 5 preceding embodiments, wherein the method comprises initializing the reference comparison parameter and wherein said initializing comprises generating the reference comparison parameter based on enrolment cough data comprising cough recordings of the targeted user.

M92. The method according to the preceding embodiment, wherein the method comprises obtaining the enrolment cough data while the targeted user is coughing.

M93. The method according to any of the 2 preceding embodiments, wherein the enrolment cough data comprise a plurality of enrolment cough audio clips and wherein generating the reference comparison parameter based on enrolment cough data comprises

computing the reference comparison parameter based on enrolment comparison parameters respectively corresponding to the enrolment cough audio clips.

It will be understood that the enrolment cough audio clips can comprise a similar data structure to the cough audio clip.

M94. The method according to any of the preceding embodiments and with the features of embodiment M86, wherein the method comprises updating the reference comparison parameter, preferably routinely.

M95. The method according to the preceding embodiment, wherein updating the reference comparison parameter is based on past cough audio clips, wherein the past cough audio clips are generated prior to the cough audio clip.

It will be understood that the past cough audio clips can comprise a similar data structure to the cough audio clip.

M96. The method according to the preceding embodiment and with the features of embodiment M15, wherein the past cough audio clips comprise one or more of the audio segments included in the cough audio data.

M97. The method according to any of the 2 preceding embodiments and with the features of embodiment M89, wherein each of the past cough audio clips used for updating the reference comparison parameter comprise a respective comparison parameter, the similarity score of which is higher than an updating threshold.

M98. The method according to any of the 3 preceding embodiments, wherein updating the reference comparison parameter is triggered after the past cough audio clips reach a predetermined number.

M99. The method according to any of the 4 preceding embodiments, wherein updating the reference comparison parameter based on past cough audio clips comprises

updating the reference comparison parameter based on comparison parameters respectively corresponding to the past cough audio clips.

M 100. The method according to any of the preceding embodiments, wherein the targeted cough detector utilizes a machine learning algorithm trained on cough data.

M101. The method according to the preceding embodiment, wherein the machine learning algorithm utilized by the targeted cough detector comprises a speaker verification algorithm.

M 102. The method according to the preceding embodiment, wherein the speaker verification algorithm is pre-trained using speech audio data to verify speaker.

M 103. The method according to any of the 2 preceding embodiments, wherein the speaker verification algorithm is further trained using cough sounds.

M104. The method according to any of the 3 preceding embodiments, wherein the method comprises configuring the speaker verification algorithm to recognize cough of the targeted user.

M105. The method according to the preceding embodiment and with the features of embodiments M94 and M91, wherein configuring the speaker verification algorithm to recognize cough of the targeted user comprises the initializing of the reference comparison parameter and the updating of the reference comparison parameter using past cough audio data.

M106. The method according to any of the preceding embodiments, wherein the method comprises generating cough metrics based on audio data containing cough of the targeted user.

M107. The method according to the preceding embodiment, wherein the cough metrics are indicative of the coughing behavior of the targeted user.

M108. The method according to any of the 2 preceding embodiments, wherein the cough metrics are indicative of a cough pattern and/or of a cough frequency.

M109. The method according to any of the 3 preceding embodiments, wherein the cough metrics are indicative of a cough-related disease, such as, a respiratory disease.

M 110. The method according to any of the 4 preceding embodiments, wherein the method comprises accumulating the cough metric over time.

M111. The method according to any of the preceding embodiments, wherein the method is a computer implemented method.

Below, system embodiments will be discussed. These embodiments are abbreviated by the letter "S" followed by a number. When reference is herein made to system embodiments, these embodiments are meant.

S1. A system for detecting cough of a targeted user, the system comprising:

a targeted cough detector configured to process a cough audio clip and to generate based thereon a result indicative of whether the cough audio clip contains a cough sound of the targeted user.

S2. The system according to any of the preceding system embodiments, wherein the system is configured to obtain source audio data, wherein the source audio data comprise the cough audio clip.

S3. The system according to the preceding embodiment, wherein the system comprises an audio recorder configured to generate the source audio data.

S4. The system according to the preceding embodiment, wherein the audio recorder is configured to continuously record the source audio data.

S5. The system according to any of the 2 preceding embodiments, wherein the audio recorder is a portable device, a wearable device or a stationary device.

S6. The system according to any of the 3 preceding embodiments, wherein the audio recorder comprises at least one auxiliary sensor configured to sense auxiliary data about a surrounding of the at least one auxiliary sensor.

S7. The system according to the preceding embodiment, wherein the auxiliary data are indicative of a humidity, pollution and/or presence of allergens in the surrounding of the at least one auxiliary sensor.

S8. The system according to any of the preceding system embodiments and with the features of embodiment S2, wherein the system comprises a cough detector configured to pre-process the source audio data to obtain cough audio data, wherein the cough audio data comprise the cough audio clip.

S9. The system according to the preceding embodiment, wherein cough detector is configured to perform the pre-processing such that the cough audio data contain less non-cough sounds than the source audio data.

S10. The system according to any of the 2 preceding embodiments, wherein the cough detector is configured to detect cough sounds.

S11. The system according to any of the 3 preceding embodiments, wherein the cough detector is configured to detect non-cough sounds,

preferably a plurality of non-cough sound types,
more preferably non-cough sound types, wherein each corresponds to a respective sound producible by a human.

S12. The system according to any of the preceding system embodiments and with the features of embodiment S2, wherein the source audio data is partitioned in audio segments.

S13. The system according to the preceding embodiment, wherein the audio segments overlap.

S14. The system according to any of the 2 preceding embodiments, wherein the audio segments comprise a constant duration, preferably at least 0.5 seconds and at most 5 seconds, more preferably at least 1 second and at most 2 seconds.

S15. The system according to any of the 3 preceding embodiments and with the features of embodiment S8, wherein the cough detector is configured to determine for each of the audio segments whether to include it in the cough audio data.

S16. The system according to the preceding embodiment, wherein the cough detector is configured to include in the cough audio data one or more of the audio segments for which the cough detector determines a positive indication on whether the audio segment contains a cough sound.

S17. The system according to any of the 2 preceding embodiments, wherein the cough detector is configured to filter out one or more of the audio segments for which the cough detector determines a positive indication on whether the audio segment contains a non-cough sound.

S18. The system according to any of the 3 preceding embodiments, wherein the cough detector is configured to determine, for each of the audio segments, a respective cough indicator indicative of whether the respective audio segment contains a cough sound and

wherein the cough detector is configured to determine for each of the audio segments whether to include it in the cough audio data based on the respective cough indicator.

S19. The system according to the preceding embodiment, wherein the cough audio data comprise for each audio segment included therein the respective cough indicator.

S20. The system according to any of the 5 preceding embodiments, wherein the cough detector is configured to determine, for each of the audio segments, a non-cough indicator indicative of whether the respective audio segment contains a non-cough sound and

wherein the cough detector is configured to determine for each of the audio segments whether to include it in the cough audio data based on the non-cough indicator.

S21. The system according to the preceding embodiment, wherein the cough audio data comprise for each audio segment included therein the respective non-cough indicator.

S22. The system according to any of the preceding system embodiments and with the features of embodiment S8,

wherein the cough detector comprises multiple stage detectors arranged sequentially such that each subsequent stage detector is configured to receive as input the output of a preceding stage detector.

S23. The system according to the preceding embodiment, wherein each subsequent stage detector is more complex than the preceding one.

S24. The system according to any of the 2 preceding embodiments, wherein one of the stage detectors is a first stage detector without any preceding stage detector and wherein one of the stage detectors is a final stage detector without any subsequent stage detector and wherein the first stage detector is configured to receive as input the source audio data and wherein the final stage detector is configured to output the cough audio data.

S25. The system according to any of the 3 preceding embodiments and with the features of embodiment S15, wherein each stage detector is configured to determine for each audio segment that it receives whether to include it in the cough audio data.

It will be understood that each stage detector can respectively make said determination as discussed in embodiments S16 to S21.

S26. The system according to any of the preceding system embodiments and with the features of embodiment S8, wherein the cough detector is configured to

generate potentially cough audio data based on the source audio data and
generate the cough audio data based on the potentially cough data.

S27. The system according to the preceding embodiment, wherein the potentially cough data contains less non-cough sounds than the source audio data.

S28. The system according to any of the 2 preceding embodiments, wherein the cough audio data contains less non-cough sounds than the potentially cough data.

S29. The system according to any of the preceding system embodiments and with the features of embodiment S8 and S26, wherein the cough detector comprises an edge detector configured to generate the potentially cough audio data based on the source audio data.

S30. The system according to the preceding embodiment and with the features of embodiment S24, wherein the edge detector is the first stage detector.

S31. The system according to any of the 2 preceding embodiments and with the features of embodiment S12, wherein the edge detector is configured to partition the source audio data in the audio segments.

S32. The system according to any of the 3 preceding embodiments and with the features of embodiment S12, wherein the edge detector is configured to determine for each of the audio segments in the source audio data whether to include it in the potentially cough audio data.

S33. The system according to the preceding embodiment, wherein the edge detector is configured to include in the potentially cough audio data one or more of the audio segments in the source audio data for which the edge detector determines a positive indication on whether the audio segment contains a cough sound.

S34. The system according to any of the 2 preceding embodiments, wherein the edge detector is configured to filter out one or more of the audio segments in the source audio data for which the edge detector determines a positive indication on whether the audio segment contains a non-cough sound.

In particular, the edge detector can be configured to filter out speech sounds.

S35. The system according to any of the 3 preceding embodiments, wherein the edge detector is configured to

determine, for each of the audio segments in the source audio data, a respective edge cough indicator indicative of whether the respective audio segment contains a cough sound and
determine, for each of the audio segments in the source audio data, whether to include it in the potentially cough audio data based on the respective edge cough indicator.

S36. The system according to the preceding embodiment, wherein the potentially cough audio data comprise for each audio segment included therein the respective edge cough indicator.

S37. The system according to any of the 5 preceding embodiments, wherein the edge detector is configured to

determine, for each of the audio segments in the source audio data, an edge non-cough indicator indicative of whether the respective audio segment contains a non-cough sound and
determine, for each of the audio segments in the source audio data, whether to include it in the potentially cough audio data based on the edge non-cough indicator.

S38. The system according to the preceding embodiment, wherein the potentially cough audio data comprises for each audio segment included therein the respective edge non-cough indicator.

S39. The system according to any of the 7 preceding embodiments, wherein the potentially cough audio data further comprises for each audio segment included therein respective segment metadata.

S40. The system according to the preceding embodiment, wherein the segment metadata comprises a recorded time of the respective audio segment.

S41. The system according to any of the 2 preceding embodiments, wherein the segment metadata comprise a user unique ID of the targeted user.

S42. The system according to any of the preceding system embodiments and with the features of embodiment S32, wherein the edge detector is configured to

generate, for each audio segment, a respective edge audio feature configured to characterize the audio segment and
determine, for each of the audio segments in the source audio data, whether to include it in the potentially cough audio data based on the respective edge audio feature.

S43. The system according to the preceding embodiment, wherein the edge audio feature is indicative of a spectrogram of the respective audio segment, wherein the spectrogram is preferably a Mel spectrogram, more preferably a log-Mel-spectrogram, even more preferably an intensity normalized log-Mel spectrogram.

S44. The system according to the preceding embodiment, wherein the edge audio feature is generated by converting the spectrogram of the respective audio segment into a two-dimensional spectrogram image, preferably a single-channel spectrogram image.

S45. The system according to any of the preceding system embodiments and with the features of embodiment S32, wherein the edge detector is configured to utilize an edge machine learning architecture, preferably based on a convolutional neural network, to determine for each of the audio segments in the source audio data whether to include it in the potentially cough audio data.

S46. The system according to the preceding embodiment, wherein the edge machine learning architecture is configured to be executed in resource constraint devices.

S47. The system according to any of the 2 preceding embodiments, wherein the edge machine learning architecture is configured to detect a visual feature in a two-dimensional image.

S48. The system according to any of the 3 preceding embodiments and with the features of embodiment S42, wherein the edge detector is configured to utilize the edge machine learning architecture to determine, for each of the audio segments in the source audio data, whether to include it in the potentially cough audio data based on the respective edge audio feature.

S49. The system according to any of the 4 preceding embodiments and with the features of embodiments S35 and/or S37, wherein the edge detector is configured to execute the edge machine learning architecture to generate the edge cough indicator and/or the edge non-cough indicator.

S50. The system according to any of the 5 preceding embodiments, wherein the edge machine learning architecture is trained using an edge training dataset, wherein the edge training dataset is generated based on a labelled audio dataset comprising cough and non-cough sounds.

S51. The system according to the preceding embodiment, wherein at least a part of the labelled audio data set is relabeled to increase labelling accuracy and/or eliminate pauses within the labelled audio dataset with a duration longer than a pause threshold.

S52. The system according to any of the 2 preceding embodiments, wherein the edge training dataset is generated further based on augmented training data, wherein the augmented training data are generated by applying one or more data augmentation techniques to the labelled audio dataset, such as, audio speed variation, audio pitch variation, noise augmentation, room impulse response, audio gain variation, time masking and/or frequency masking.

S53. The system according to any of the preceding system embodiments and with the features of embodiment S8 and S26, wherein the cough detector comprises a cloud detector configured to generate the cough audio data based on the potentially cough audio data.

S54. The system according to the preceding embodiment and with the features of embodiment S24, wherein the cloud detector is the final stage detector.

S55. The system according to any of the 2 preceding embodiments and with the features of embodiment S29, wherein the cloud detector comprises more computational resources than the edge detector.

S56. The system according to any of the 3 preceding embodiments and with the features of embodiment S12, wherein the cloud detector is configured to determine, for each of the audio segments in the potentially cough audio data whether to include it in the cough audio data.

S57. The system according to the preceding embodiment, wherein the cloud detector is configured to include in the cough audio data one or more of the audio segments in the potentially cough audio data for which the cloud detector determines a positive indication on whether the audio segment contains a cough sound.

S58. The system according to any of the 2 preceding embodiments, wherein the cloud detector is configured to filter out one or more of the audio segments in the potentially cough audio data for which the cloud detector determines a positive indication on whether the audio segment contains a non-cough sound.

S59. The system according to any of the 3 preceding embodiments, wherein the cloud detector is configured to

determine, for each of the audio segments in the potentially cough audio data, a respective cloud cough indicator indicative of whether the respective audio segment contains a cough sound and
determine, for each of the audio segments in the potentially cough audio data, whether to include it in the cough audio data based on the respective cloud cough indicator.

S60. The system according to the preceding embodiment, wherein the cough audio data comprises for each audio segment included therein the respective cloud cough indicator.

S61. The system according to any of the 5 preceding embodiments wherein the cloud detector is configured to

determine, for each of the audio segments in the potentially cough audio data, a cloud non-cough indicator indicative of whether the respective audio segment contains a non-cough sound and
determine, for each of the audio segments in the potentially cough audio data, whether to include it in the cough audio data based on the cloud non-cough indicator.

S62. The system according to the preceding embodiment, wherein the cough audio data comprises for each audio segment included therein the respective cloud non-cough indicator.

S63. The system according to any of the 7 preceding embodiments, wherein the cough audio data further comprises for each audio segment included therein respective segment metadata.

S64. The system according to the preceding embodiment, wherein the segment metadata comprise a recorded time of the respective audio segment.

S65. The system according to any of the 2 preceding embodiments, wherein the segment metadata comprise a user unique ID of the targeted user.

S66. The system according to any of the preceding system embodiments and with the features of embodiment S56, wherein cloud detector is configured to

generate, for each audio segment, a respective cloud audio feature configured to characterize the audio segment and
determine, for each of the audio segments in the potentially cough audio data, whether to include it in the cough audio data is based on the respective cloud audio feature.

S67. The system according to the preceding embodiment, wherein the cloud audio feature is indicative of a spectrogram of the respective audio segment, wherein the spectrogram is preferably a Mel spectrogram, more preferably a log-Mel-spectrogram, even more preferably an intensity normalized log-Mel spectrogram.

S68. The system according to any of the preceding system embodiments and with the features of embodiment S56, wherein the cloud detector is configured to utilize a cloud machine learning architecture, preferably based on Transformer models, to determine for each of the audio segments in the potentially cough audio data whether to include it in the cough audio data.

S69. The system according to the preceding embodiment, wherein the cloud machine learning architecture is configured to be executed in resource extensive devices.

S70. The system according to any of the 2 preceding embodiments and with the features of embodiment S66, wherein the cloud detector is configured to utilize the cloud machine learning architecture to determine, for each of the audio segments in the potentially cough audio data, whether to include it in the cough audio data based on the respective cloud audio feature.

S71. The system according to any of the 4 preceding embodiments and with the features of embodiments S59 and/or S61, wherein the cloud detector is configured to execute the cloud machine learning architecture to generate the cloud cough indicator and/or the cloud non-cough indicator.

S72. The system according to any of the 5 preceding embodiments, wherein the cloud machine learning architecture is trained using a cloud training dataset, wherein the cloud training dataset is generated based on a labelled audio dataset comprising cough and non-cough sounds.

S73. The system according to the preceding embodiment, wherein at least a part of the labelled audio data set is relabeled to increase labelling accuracy and/or eliminate pauses within the labelled audio dataset with a duration longer than a pause threshold.

S74. The system according to any of the 2 preceding embodiments, wherein the cloud training dataset is generated further based on augmented training data, wherein the augmented training data are generated by applying one or more data augmentation techniques to the labelled audio dataset, such as, audio speed variation, audio pitch variation, noise augmentation, room impulse response, audio gain variation, time masking and/or frequency masking.

S75. The system according to any of the 3 preceding embodiments and with the features of embodiment S50, wherein the edge training dataset and the cloud training dataset are the same.

S76. The system according to any of the preceding system embodiments and with the features of embodiment S29, wherein the system comprises an edge system and wherein the edge detector is part of the edge system.

S77. The system according to the preceding embodiment and with the features of embodiment S3, wherein the edge system comprises the audio recorder.

S78. The system according to any of the 2 preceding embodiments, wherein the edge system comprises an edge communication device.

S79. The system according to the preceding embodiment and with the features of embodiment S53, wherein the edge communication device is configured to transmit the potentially cough audio data to the cloud detector.

S80. The system according to any of the preceding system embodiments, wherein the system comprises a cloud system and wherein the target cough detector is part of the cloud system.

S81. The system according to the preceding embodiment and with the features of embodiment S53, wherein the cloud system comprises the targeted cough detector.

S82. The system according to any of the preceding system embodiments and with the features of embodiment S53, wherein the cloud detector is configured to transmit the cough audio data to the targeted cough detector.

S83. The system according to any of the preceding system embodiments, wherein the targeted cough detector is configured to differentiate cough of the targeted user from cough of other individuals.

S84. The system according to any of the preceding system embodiments and with the features of embodiment S12, wherein the cough audio clip comprises one or more of the audio segments.

S85. The system according to any of the preceding system embodiments and with the features of embodiment S15, wherein the cough audio clip comprises one or more of the audio segments included in the cough audio data.

S86. The system according to any of the preceding system embodiments, wherein the targeted cough detector is configured to

> compute a comparison parameter corresponding to the cough audio clip,
> compare the comparison parameter with a reference comparison parameter corresponding to the targeted user,
> generate the result based on the comparison.

S87. The system according to the preceding embodiment, wherein the comparison parameter and the reference comparison parameter comprise the same structure.

S88. The system according to any of the 2 preceding embodiments, wherein the comparison parameter is a vector representation, such as, an embedding, of the cough audio clip.

S89. The system according to any of the 3 preceding embodiments, wherein the targeted cough detector is configured to compute a similarity score between the comparison parameter and the reference comparison parameter.

S90. The system according to the preceding embodiment, wherein the result comprises

a positive indication that the cough audio clip contains a cough sound of the targeted user if the similarity score is higher than a similarity threshold and

a negative indication otherwise.

S91. The system according to any of the 5 preceding embodiments, wherein the targeted cough detector is configured to initialize the reference comparison parameter by generating the reference comparison parameter based on enrolment cough data comprising cough recordings of the targeted user.

S92. The system according to the preceding embodiment, wherein the enrolment cough data are obtained while the targeted user is coughing.

S93. The system according to any of the 2 preceding embodiments, wherein the enrolment cough data comprise a plurality of enrolment cough audio clips and wherein the targeted cough detector is configured to compute the reference comparison parameter based on enrolment comparison parameters respectively corresponding to the enrolment cough audio clips.

It will be understood that the enrolment cough audio clips can comprise a similar data structure to the cough audio clip.

S94. The system according to any of the preceding system embodiments and with the features of embodiment S86, wherein the targeted cough detector is configured to update the reference comparison parameter, preferably routinely.

S95. The system according to the preceding embodiment, wherein the targeted cough detector is configured to update the reference comparison parameter based on past cough audio clips, wherein the past cough audio clips are generated prior to the cough audio clip.

It will be understood that the past cough audio clips can comprise a similar data structure to the cough audio clip.

S96. The system according to the preceding embodiment and with the features of embodiment S15, wherein the past cough audio clips comprise one or more of the audio segments included in the cough audio data.

S97. The system according to any of the 2 preceding embodiments and with the features of embodiment S89, wherein each of the past cough audio clips used for updating the reference comparison parameter comprise a respective comparison parameter, the similarity score of which is higher than an updating threshold.

S98. The system according to any of the 3 preceding embodiments, wherein the update of the reference comparison parameter is triggered after the past cough audio clips reach a predetermined number.

S99. The system according to any of the 4 preceding embodiments, wherein the targeted cough detector is configured to update the reference comparison parameter based on comparison parameters respectively corresponding to the past cough audio clips.

S100. The system according to any of the preceding system embodiments, wherein the targeted cough detector is configured to utilize a machine learning algorithm trained on cough data.

S101. The system according to the preceding embodiment, wherein the machine learning algorithm utilized by the targeted cough detector comprises a speaker verification algorithm.

S102. The system according to the preceding embodiment, wherein the speaker verification algorithm is pre-trained using speech audio data to verify speaker.

S103. The system according to any of the 2 preceding embodiments, wherein the speaker verification algorithm is further trained using cough sounds.

S104. The system according to any of the 3 preceding system embodiments, wherein the speaker verification algorithm is configured to recognize cough of the targeted user.

S105. The system according to the preceding embodiment and with the features of embodiments S94 and S91, wherein the speaker verification algorithm is configured to recognize cough of the targeted user by initializing the reference comparison parameter and by updating the reference comparison parameter using past cough audio data.

S106. The system according to any of the preceding system embodiments, wherein the targeted cough detector is configured to generate cough metrics based on audio data containing cough of the targeted user.

S107. The system according to the preceding embodiment, wherein the cough metrics are indicative of the coughing behavior of the targeted user.

S108. The system according to any of the 2 preceding embodiments, wherein the cough metrics are indicative of a cough pattern and/or of a cough frequency.

S109. The system according to any of the 3 preceding embodiments, wherein the cough metrics are indicative of a cough-related disease, such as, a respiratory disease.

S110. The system according to any of the 4 preceding embodiments, wherein the targeted cough detector is configured to accumulate the cough metric over time.

Sill. The system according to any of the preceding system embodiments, wherein the system is configured to carry out the method according to any of the preceding method embodiments.

Below further method embodiments will be discussed.

M112. The method according to any of the preceding method embodiments, wherein the method is carried out by the system according to any of the preceding system embodiments.

Below, further embodiments will be discussed.

C1. A computer program product comprising instructions which, when the program is executed by a system according to any of the preceding system embodiments, cause the system to carry out the method according to any of the preceding method embodiments.

C2. A computer-readable storage medium comprising instructions which, when executed by a system according to any of the preceding system embodiments, cause the system to carry out the method according to any of the preceding method embodiments.

**Brief description of the drawings**

| | |
|---|---|
| **Fig. 1** | depicts a high-level overview of a system to detect cough of a targeted user; |
| **Fig. 2** | depicts a more detailed overview of the system to detect cough of a targeted user; |
| **Fig. 3** | depicts a block diagram of an edge system; |
| **Fig. 4** | depicts a flowchart for edge cough detection; |
| **Figs. 5a-5b** | depicts a more detailed flowchart for edge cough detection; |
| **Fig. 6** | depicts a flowchart for cloud cough detection; |
| **Fig. 7** | illustrates a computation of a comparison parameter; |
| **Fig. 8** | illustrates a computation of a reference comparison parameter; |
| **Fig. 9** | is a flowchart for determining whether a cough audio clip contains a cough sound of the targeted user; |
| **Figs. 10a-10b** | depict a flowchart of computing and updating a reference comparison parameter. |

**Detailed description of the drawings**

In the following, exemplary embodiments of the invention will be described, referring to the figures. These examples are provided to give further understanding of the invention, without limiting its scope.

In the following description, a series of features and/or steps are described. The skilled person will appreciate that unless explicitly required and/or unless required by context, the order of features and steps is not critical for the resulting configuration and its effect. Further, it will be apparent to the skilled person that irrespective of the order of features and steps, the presence or absence of time delay between steps can be present between some or all of the described steps.

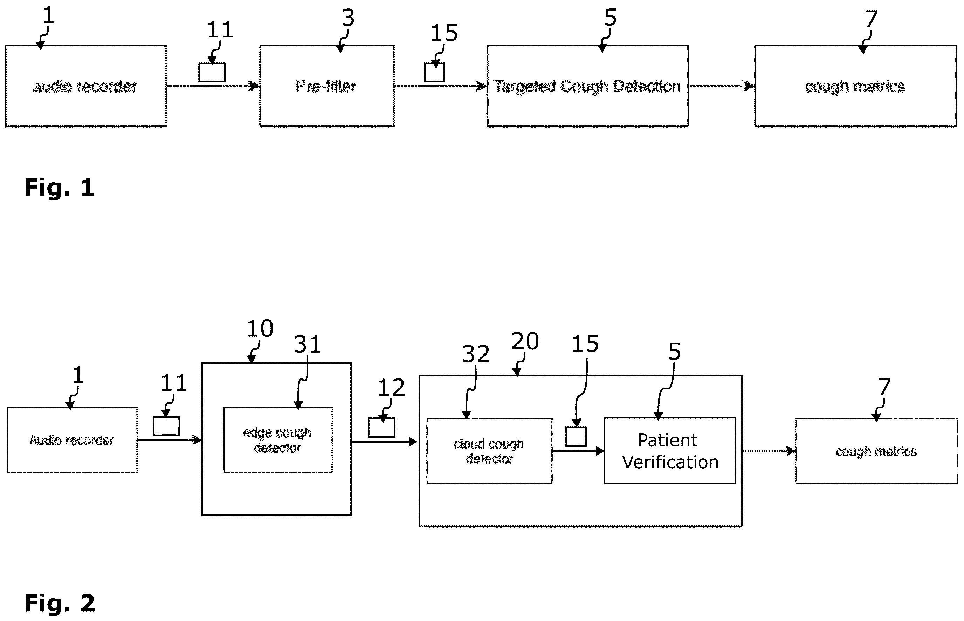

The present invention can detect cough of a targeted user from audio recordings and generate cough metrics. The audio metrics can be used to detect any change in cough pattern/frequency which could aid healthcare providers in diagnosing various respiratory diseases. To achieve this, the present invention can comprise a series of Machine Learning (ML) based audio processing modules as shown in Fig 1.

**Fig. 1** depicts a high-level overview of a system to detect cough of a targeted user.

The system can comprise an audio recorder 1. The audio recorder 1 can be configured to continuously record audio in a suitable format for downstream tasks to thereby generate source audio data 11. The audio recorder 1 can be a portable device, such as, a smartphone or a smartwatch, or it can be a stationary device like smart-home devices or it can be a customized hardware. Furthermore, the audio recorder 1 can send data related to the user's surrounding like humidity, pollution, presence of allergens and so on which can be indicative of the user's coughing behavior.

The system can comprise a pre-filter 3. The pre-filter 3 can also be referred to as a preprocessor 3 or as a cough detector 3. The pre-filter 3 can be configured to pre-process the source audio data 11 to obtain cough audio data 15.

The pre-filter 3 can be configured to separate out cough-like sound from the rest. This can facilitate the subsequent targeted cough detection module 5 to function reliably. Moreover, the pre-filter 3 can filter-out any other undesired sound, like speech, produced around the targeted user's vicinity. This can protect the targeted user's privacy. The pre-filter 3 (i.e., cough detector 3) can comprise a single or a series of modules and can be implemented at edge or in hybrid (i.e., edge + cloud) settings.

The system can further comprise a targeted cough detector 5. The targeted cough detector 5 can separate out the targeted user's cough from the rest. The targeted cough detector 5 may comprise a machine learning model trained on corpus of cough data.

The result of the targeted cough detector 5 can be accumulated over time and/or can be used to obtain cough metrics 7. The cough metrics 7 can provide various insights to the coughing behavior of the user which can help healthcare providers to effectively diagnose various the cough related diseases.

**Fig. 2** depicts a more detailed overview of the system to detect cough of a targeted user. In particular, Fig. 2 provides more implementation details.

As illustrated in Fig. 2, the system can comprise an edge system 10 and a cloud system 20. The cloud system 20 can be provided at a remote location from the edge system 10. Moreover, the cloud system 20 can be configured to serve multiple edge systems 10. In such embodiments, the edge system 10 can comprise a respective targeted cough detector 5 for each targeted user.

On the other hand, the edge system 10 can be provided at the vicinity of the targeted user. Moreover, one or more edge systems 10 may be specific to the targeted user. Thus, in case of multiple edge systems 10, one or more edge systems 10 can correspond to a respective targeted user. In other words, each of the edge systems 10 can be assigned to a respective targeted user, wherein, e.g., multiple edge systems may be assigned to the same targeted user. For example, the same targeted user can use multiple edge devices, e.g., to cover each room in their home.

Furthermore, as illustrated in Fig. 2, the system and in particular the cough detector 3 can comprise an edge cough detector 31 and a cloud cough detector 32. The edge cough detector 31 can be part of the edge system 10 and the cloud cough detector 32 can be part of the cloud system 20. The edge cough detector 31 can be configured to obtain the source audio data 11 and to generate based thereon potentially cough audio data 12, such that, the potentially cough audio data 12 can comprise less non-cough sounds than the source audio data. The cloud cough detector 32 can be configured to obtain the potentially cough audio data 12 and to generate based thereon cough audio data 15, such that, the cough audio data comprise less non-cough sounds than the potentially cough audio data. The cough audio data 15 can be provided to the target cough detector 5 (which in the figure is referred to as Patient Verification, as it verifies whether the cough corresponds to the targeted user, i.e., the patient or not).

Moreover, while in Fig. 2, the audio recorder is illustrated as being external to the edge system 10, it will be understood that the edge system 10 may comprise the audio recorder 1.

Further still, while in Fig. 2, the cloud cough detector 32 and the targeted cough detector 5 are depicted as being part of the same cloud system 20, it will be understood that the cloud cough detector 32 and the targeted cough detector 5 may also be part of respective and separate cloud systems (not shown).

For the sake of brevity, the edge cough detector 31 and the cloud cough detector 32 can be referred to as an edge detector 31 and cloud detector 32, respectively.

Below, each system component will be discussed in more detail.

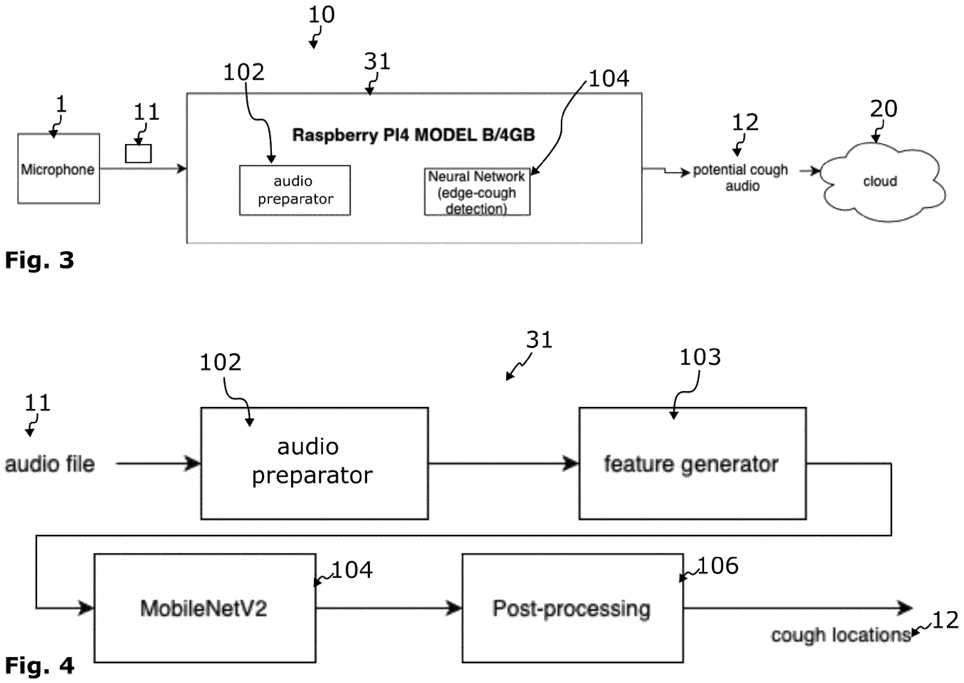

**Fig. 3** illustrates an edge system 10. The edge system 10 can be configured to record, process, filter and transmit the recorded cough to the cloud system 20 for further processing. The edge system 10 can be a custom hardware solution. It can provide a course pre-filter for undesired sounds like speech, laugh etc.

The edge system 10 may comprise the edge cough detector 31, which can be a computer, such as, a single board computer, e.g., a Raspberry Pi-4. An audio recorder 1, e.g., a microphone 1, can be interfaced with edge cough detector 31.

The edge detector 31 may comprise an edge audio preparator 102 and an edge machine learning architecture 104 which may be a neural network edge cough detection module 104. The edge detector 31 may be configured to generate, from the raw audio signal, a digital audio file. This can be achieved by various transformations like Analog to Digital conversion, digital signal filtering, amplification and/or normalization. The resulting signal can be saved in a lossless audio format such as a 16bit PCM Flac file, with sampling frequency 32 kHz. It will be understood that this is only exemplary and that other file formats or sampling frequencies may be used.

The edge system 10, in particular the cough detector 31, may generate the potentially cough audio data 12. The edge system 10 can transmit the potentially cough audio data 12 to the cloud system 20, in particular, to the cloud cough detector 32 (see Fig. 2).

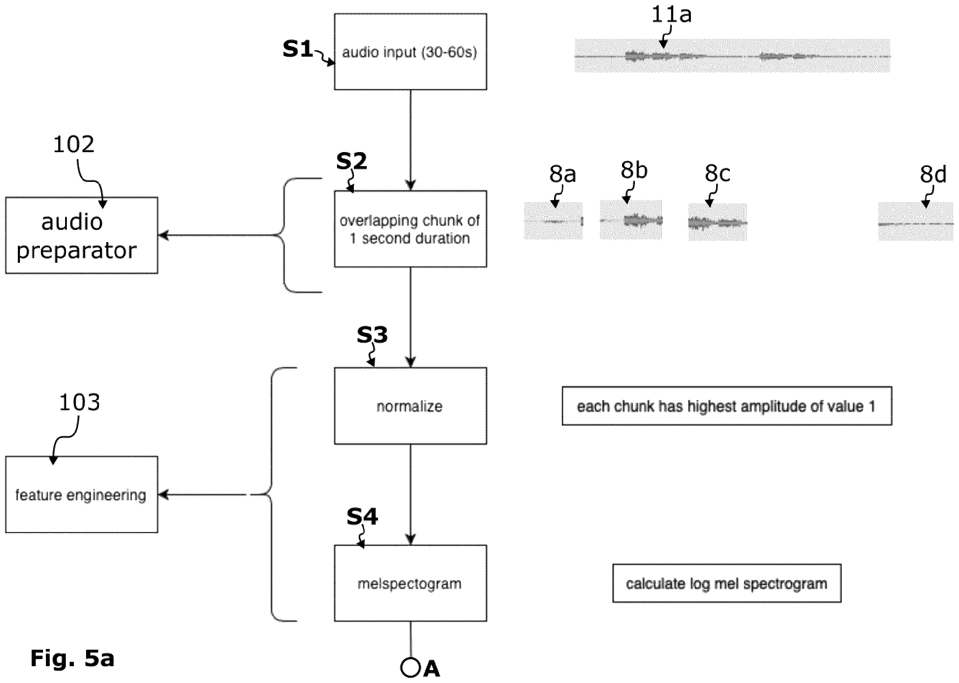

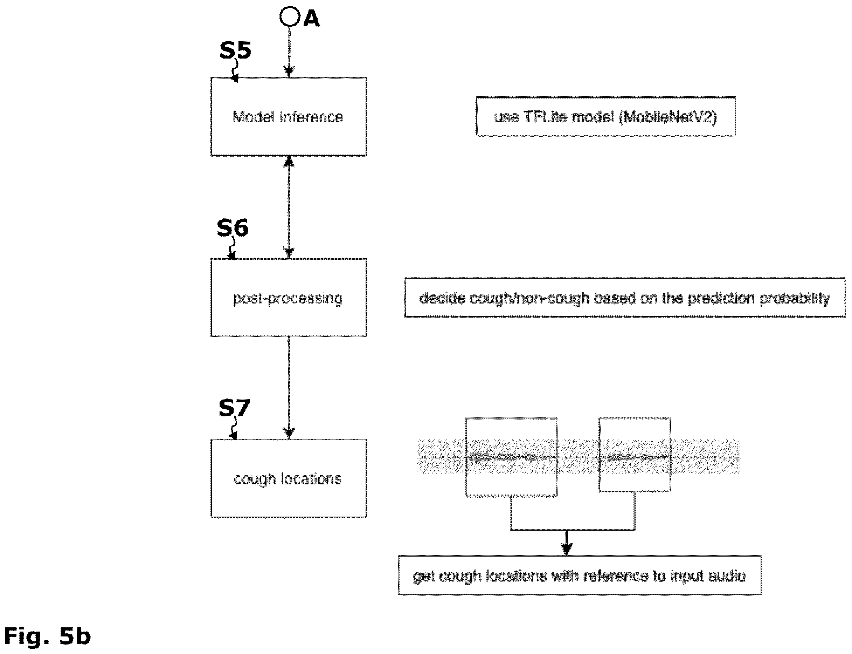

Below with respect to Figures 4, 5a and 5b the edge cough detector 31, also referred to as an edge detector 31, will be discussed.

**Fig. 4** depicts a more detailed block diagram of the edge cough detector 31 and Figs. 5a to 5b depicts the logical flow of the edge cough detector 31. It will be understood that Figs. 5a to 5b, each show a part of a flowchart depicting the logical flow of the edge cough detector 31, said parts joinable at point A.

Generally, the audio file, i.e., source audio data 11, can be fed to the edge-cough detector 31, which can be a convolutional neural network (CNN) based neural network. The cough detector 31 can take the source audio data 11 as input and can predict the presence of cough. It can subsequently provide the time location of the potential cough in the input file.

Thus, in S1 the edge cough detector 31 can obtain the source audio data 11, which can for example be an audio file 11 with a length of, e.g., 30-60s. Fig. 5a, depicts an audio waveform 11a of the source audio data 11.

The edge detector 31 can comprise an edge audio preparator 102. In S2, the edge audio preparator 102 can take the source audio data 11 as input and can apply various preprocessing steps. The source audio data 11 can be loaded with the sampling rate of 16 kHz and using single channel. The source audio data 11 can be loaded with lower sampling rate in order to save memory and compute resources. At first, the source audio data 11 can be split into audio segments 8, which can also be referred to as audio chunks 8. The audio segments 8 can be overlapping chunks 8 and can comprise a constant duration of, e.g., 1 second duration.

The edge detector 31 can comprise can comprise an edge feature generator 103. In S3 and S4, each audio chunk 8 can be fed to the edge feature generator 103, which can generate respective spectrograms, preferably, Mel spectrogram features, more preferably log-Mel spectrograms. The spectrograms can be normalized using Per-Channel Energy Normalization (PCEN) to obtain channel normalized spectrograms, preferably channel normalized Mel spectrograms.

Each spectrogram can represent both time and frequency properties of the audio simultaneously. Each log-Mel spectrogram can be a float32 tensor representing the logarithm of the respective Mel-spectrogram of the respective audio chunk 8. These are the audio features passed into the neural network edge cough detection module 104.

Each spectrogram can have a two-dimensional shape, with one dimension (e.g., y-axis) indicative of the number of spectrogram frequency bins and the other dimension (e.g., x-axis) indicative of the number of spectrogram temporal frames. The number of spectrogram temporal frames can be the number of frames produced by sliding on a waveform of an audio chunk 8 a spectrogram analysis window. The number of spectrogram frequency bins can represent the number of Mel bins. The optimum value of the spectrogram bins and frames can be determined based on feature engineering. For each audio chunk, a respective spectrogram of predetermined dimensions can be generated. The spectrogram can be converted to a greyscale image and can be normalized for scale consistency. The greyscale image, preferably normalized, can be supplied to the neural network edge cough detection module 104.

That is, in S3 and S4 an audio feature can be generated for each audio segment 8. The audio feature can be a spectrogram, preferably a normalized log-Mel spectrogram. In S5 the audio feature can be provided to the neural network edge cough detector 104, which can for example utilize the MobileNetV2 model.

It will be understood that the above dimensions for the spectrograms are exemplary.

The neural network edge cough detection module 104 can be a convolutional neural network architecture configured to perform efficiently on power limited mobile/edge devices. For example, the neural network edge cough detection module 104 can comprise MobileNetV2, the architecture details of which are described in the paper: Sandler, Mark, et al. "MobileNetV2: Inverted Residuals and Linear Bottlenecks" (2019), https://doi.org/10.48550/ arXiv.1801.04381.

In short, MobileNetV2 is based on the concept of an inverted residual structure where the residual connections are between the bottleneck layers. The intermediate expansion layer uses lightweight depth-wise convolutions to filter features as a source of non-linearity. All in all, the architecture of MobileNetV2 contains the initial fully convolution layer with 32 filters, followed by 19 residual bottleneck layers.

In particular, the neural network edge cough detection module 104 can comprise a pretrained model of MobileNetV2, which is trained in the ImageNet dataset, introduced by: Pytorch Team. "MOBILENET V2" https:// pytorch.org/hub/pytorch vision mobilenet v2/.

The ImageNet dataset is an image database by Stanford Vision Lab, Stanford University, Princeton University retrievable from https://www.image-net.org.

Said pretrained model can be used as a starting point and can be fine-tuned using audio dataset comprising of cough and non-cough sounds.

Furthermore, the input and output layers of MobileNetV2 can be modified. In particular, the input layer of the model can be configured to accept single channel (e.g., greyscale) image instead of conventional RGB image. Additionally, the fully connected output layer can be configured to contain 2 output neurons which can output predictions for cough and non-cough respectively.

The following dataset can be used to train the neural network edge cough detection module 104 for cough detection:

1. Fonseca, E. et al. "FSD50K: An Open Dataset of Human-Labeled Sound Events" (2022), https://arxiv.org/abs/ 2010.00475. Freesound Dataset 50k (FSD50K) contains various sounds that can be produced by a human user, such as coughing, laughing, speaking, moaning, sneezing, throat clearing, sighing etc. It is an open dataset of human-labeled sound events containing 51,197 Freesound clips unequally distributed in 200 classes drawn from the AudioSet Ontology. Furthermore, the dataset can be re-labelled to obtain high-quality label. Additionally, the re-labelling may also eliminate the long pauses (silence/background) in between the audio events within a same file for higher labelling accuracy. The re-labelled audio files can be used for training.

2. Orlandic L. et al, "The COUGHVID crowdsourcing dataset, a corpus for the study of large-scale cough analysis algorithms" (2021), https://www.nature.com/articles/s41597-021-00937-4. The COUGHVID dataset provides over 30,000 crowdsourced cough recordings representing a wide range of subject ages, genders, geographic locations, and COVID-19 statuses. Audio files whose cough probability are greater than 70% are used for the model training.

3. Propriety Data which can comprise audio data collected from test subjects. Said data can be labelled and used for training.

4. VoxCeleb2: Son Chung, J. et al. "VoxCeleb2: Deep Speaker Recognition" (2018), https://arxiv.org/abs/1806.05622. VoxCeleb2 is a large-scale speaker recognition dataset obtained automatically from open-source media which consists of over a million utterances from over 6000 speakers. For training, a part of the audio files of VoxCeleb2 is sampled with equal number of male and female speakers.

During training, various data augmentation techniques can be randomly applied to artificially enlarge the training dataset. The augmentation techniques can include:

1. Audio speed variation.
2. Audio pitch variation.
3. Noise augmentation. For example, the MUSAN dataset introduced in Snyder, D. "MUSAN: A Music, Speech, and Noise Corpus" (2015), https://arxiv.org/abs/1510.08484 can be used. The recording device's fan sound can be used to append noise to the dataset.
4. Room impulse response. This augmentation technique can be used to artificially simulate various room environments based on impulse response dataset. For this the dataset introduced in Traer, J et al. "Statistics of natural reverberation enable perceptual separation of sound and space" (2016), https://mcdermottlab.mit.edu/Reverb/IR Survey.html can be used.
5. Audio gain variation.
6. Time masking.
7. Frequency masking.

The neural network edge cough detection module 104 can be trained for a predetermined maximum number of epochs with a predetermined batch size and a predetermined initial learning rate. A learning rate scheduler can be implemented which can halve the learning rate if the validation error plateaus within a predetermined number of consecutive epochs. Furthermore, early stopping criteria can be implemented for the training which can stop the training if the validation error does not decrease after a predetermined number of consecutive epochs.

Moreover, cross entropy loss and Adam optimizer can be used for learning the model weights. The Adam optimizer refers to the optimization technique introduced in:

Kingma, D. P. et al. "Adam: A Method for Stochastic Optimization" (2017), https://arxiv.org/abs/1412.6980.

The input audio files 11 can be of arbitrary length. Also, the dataset can comprise file-level labels, hence, it can be challenging to ascertain the location of the audio event within the long audio files. To alleviate this, the maximum of the predictions of all audio chunks within an audio file can be selected to obtain the final cough prediction. During training, the final model is selected based on lowest validation error.

Table I and Table II indicate statistics of training and testing the edge cough detector 31, in particular the neural network edge cough detection module 104.

| Table I | | |
|---|---|---|
| Data Type | Training | testing |
| cough | 10984 | 2628 |
| non cough | 33520 | 8515 |
| total | 44504 | 11143 |

| Table II | |
|---|---|
| Metric | Values [%] |
| Accuracy | 99.48 |
| F1 | 98.50 |
| Precision | 99.24 |
| Recall | 98.49 |

Table I indicates the number of audio files 11 used for training and testing the edge cough detector 31, said files divided in cough and non-cough as indicated by the first column "Data Type". The second column "Training" indicates the number of files used for training and validation of the edge cough detector 31 and the third column indicates the number of files used for testing the edge cough detector 31.

Table II indicates the test results obtained by the best performing neural network edge cough detection module 104. It will be understood that in Table II, F1 refers to the F1 scope which is calculated as the harmonic mean of the precision and recall scores.

In S5 the neural network edge cough detection module 104 can generate cough indicators indicative of the presence of cough sound on the audio segments respectively. In S5, the neural network edge cough detection module 104 may also generate non-cough indicators indicative of the presence of non-cough sound on the audio segments respective.

In S6 based on the cough and/or non-cough indicators an edge post processing module 106 can decide presence of cough and/or of non-cough in each audio segment.

In S7 cough locations in the source audio data can be determined. Once the cough is detected in the audio segment, their respective locations with respect to the original audio can be determined by a mapping function. If audio segments wherein cough is detected are overlapping, then their locations can be merged and the cough clip can be extracted and send to the cloud for further processing.

Overall, the edge system 10 can monitor the targeted user continuously and can send out any detected cough to the cloud system 20. That is, the edge system 10 can send potentially cough data 12 to the cloud system 20. Specifically, the audio snippet containing the cough along with other metadata (e.g., recorded time, prediction probability, user unique ID) can be sent out.

Communication protocols, such as, Internet-of-Things communication protocols, can be implemented, for the data transmission between the edge system 10 and the cloud system 20.

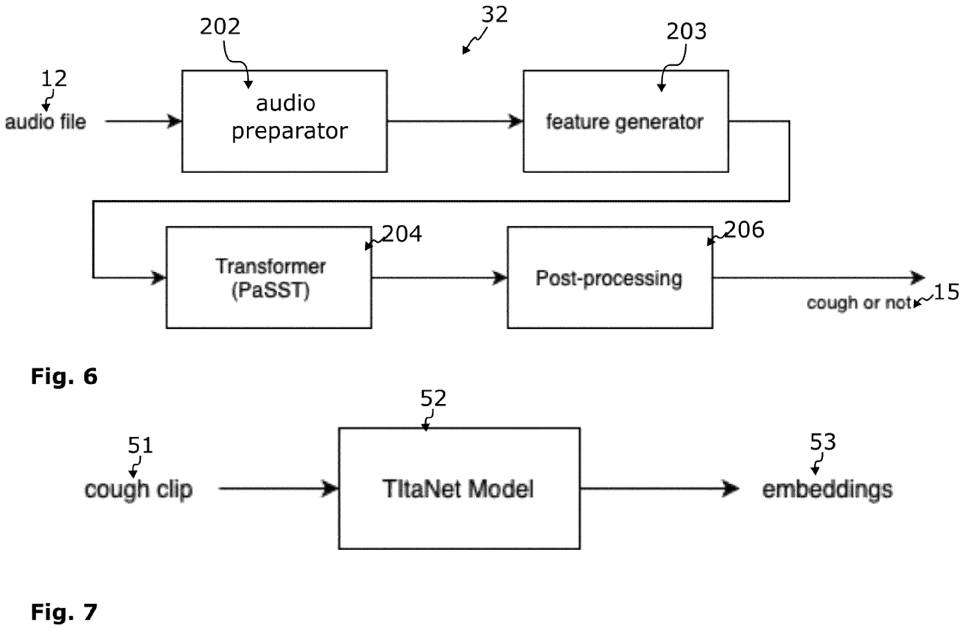

**Fig. 6** depicts an overview of the cloud cough detector 32, which for brevity can also be referred to as the cloud detector 32.

The potential cough data 12 can be sent out to the cloud detector 32 for further processing. The cloud detector 32 can provide large computational capabilities as well as methods to flexibly distribute the results to the concerned clinicians.

The edge cough detector 31 can efficiently execute only small neural network models, which may not be powerful enough to separate out undesired sounds like speech, laugh and cry with high accuracy. The cloud cough detector 32 can be utilized to eliminate potential false positives that may be present in the potentially cough data 12. The cloud cough detector 32 may be based Transformer models. Though computationally expensive, Transformer models can show state-of-the-art performance in various vision as well as audio tasks.

The potential cough audio data 12 transmitted from the edge cough detector 31 can be further filtered with the cloud cough detector 32. The audio can be loaded into the cloud cough detector 32 with the sampling rate of 16 kHz and using single channel.

The cloud cough detector 32 can comprise a cloud audio preparator 202 which can take as input the potentially cough audio data 12 received from the edge cough detector 31 and can apply various preparation steps. The cloud audio preparator 202 may apply on the potentially cough audio data 12 similar preparation techniques as discussed with respect to the edge audio preparator 102. For example, the cloud audio preparator 202 may split the potentially cough audio data 12 into audio segments 8, which can also be referred to as audio chunks 8. The audio segments 8 can be overlapping chunks 8 and can comprise a constant duration of, e.g., 1 second duration.

The cloud cough detector 32 can comprise a cloud feature generator 203 which can generate log-Mel-spectrogram features from each of the audio chunks 8 in the potentially cough audio data 12. The spectrogram generation parameters can be adapted to cough domain. In one example, spectrogram generation parameters can include, at least one of: number of FFT points, window size, hop size (i.e., number of samples by which consecutive windows are shifted), number of Mel bins, maximum frequency and minimum frequency. Optimum values for the spectrogram generation parameters can be determined based on feature engineering.

The cloud cough detector 32 can comprise an audio Transformer model 204, which can for example, be the audio Transformer model introduced in:

Koutini, K. et al. "Efficient Training of Audio Transformers with Patchout", (2022), https://arxiv.org/abs/2110.05069.

The audio Transformer model 204 can be trained on ImageNet dataset.

The audio Transformer model 204 can be trained using a large corpus of audio data (e.g., AudioSet).

The audio Transformer model 204 can be adapted to the audio domain using Patchout Fast Spectrogram Transformer (PASST) technique, as taught by Koutini, K. et al, and can achieve state-of-the-art results in various audio tasks.

The trained weights for the PASST model be taken from Koutini, K. et al and can be further fine-tuned for cough detection task.

The same datasets discussed above in relation to the neural network edge cough detection module 104 can be used for training the audio Transformer model 204.

During training, various data augmentation techniques can be randomly applied to artificially enlarge the training dataset, as discussed above.

The audio Transformer model 204 can be trained for predetermined maximum number of epochs with a predetermined batch size and a predetermined initial learning rate. A learning rate scheduler can be implemented which can halve the learning rate if the validation error plateaus within a predetermined number of consecutive epochs. Furthermore, early stopping criteria can be implemented for the training which can stop the training if the validation error does not decrease after a predetermined number of consecutive epochs.

Moreover, cross entropy loss and Adam optimizer can be used for learning the model weights.

The cloud cough detector 32 can comprise a cloud post-processing module 206 which can output an output vector of length of 2 indicating the probability of cough and non-cough sound in the audio clip.

Table III indicates statistics of training and testing the cloud cough detector 32. In particular, it indicates the number of audio files 11 used for training and testing the cloud cough detector 32, said files divided in cough and non-cough as indicated by the first column "Data Type". The second column "Training" indicates the number of files used for training the edge cough detector 31 and the third column indicates the number of files used for testing the edge cough detector 31.

| Table III | | |
|---|---|---|
| **Data Type** | **Training** | **Testing** |
| cough | 19524 | 3208 |
| non cough | 42939 | 4901 |
| total | 62463 | 8109 |

The best performing model obtained an F1 score of 95.5% on the above-mentioned test set.

The cloud cough detector 32 can determine presence of cough in the audio snippet(s) obtained from the edge detector 31 with higher confidence.

Below the targeted cough detector 5 will be discussed. The processing performed by the targeted cough detector 5 can also be referred to as patient verification algorithm.

The targeted cough detector 5 can use a pre-trained model called TitaNet introduced in Koluguri, N. R. et al. "TitaNet: Neural Model for speaker representation with 1D Depth-wise separable convolutions and global context" (2021), https://arxiv.org/abs/2110.04410.

TitaNet is trained for speaker verification using various speech dataset including VoxCeleb1, VoxCeleb2, SRE etc. It is trained on more than 16000 speakers with more than 3000 hours of audio. The TitaNet model has achieved state-of-the-art performance on various benchmarks datasets.

In particular, the TitaNet-L variant from NVIDIA NeMo (obtainable in: https://docs.nvidia.com/nemo-framework/user-quide/latest/nemotoolkit/asr/speaker_recognition/models.html#id1), which is a conversational AI toolkit built for researchers from industry and academia, can be used.

Although the model was developed for speech tasks, it can be fine-tuned for targeted user cough verification using cough sound.

The pre-trained model from TitaNet can be fine-tuned for detecting cough of a targeted user, i.e., for a patient verification task. To achieve this, a cough audio dataset can be utilized. Said cough audio dataset can be used to fine-tune the speaker verification algorithm (e.g., the pre-trained model from TitaNet) for cough detection of the targeted user. The cough audio dataset can be collected, e.g., over a 3-day period, using an audio recorder placed at the targeted users' residential environment.

The cough audio dataset collected at the residential environment may contain non-cough sounds and cough sounds from other users. Therefore, multiple cough detection algorithms can be applied to significantly decrease the occurrence of non-cough sounds in the cough audio dataset. For example, the dataset can be processed using the edge cough detector 31 and the cloud cough detector 32. In particular, the edge cough detector 31 can be used to obtain cough audio clips with high recall. Then, these audio clips can be fed to the cloud cough detector 32 to filter out the false positives.

The first step to initiate the process of dataset collection is to enroll the cough of the targeted user. The targeted user can provide cough recordings, e.g., 5 to 20 cough recordings, preferably 10 to 15 cough recording. This can facilitate reliable computation of the cough pattern of the targeted user.

To remove the cough from other users in the dataset, the pretrained speaker verification model from NVIDIA NeMo can be used with the patient enrolment files to obtain high confidence targeted user's coughs from the dataset. Although the NVIDIA NeMo is trained on speech instead of cough, this initial cleaning step can help to achieve a high-quality dataset. The dataset obtained is used for fine-tuning, validation and testing.

The procedure can be summarized with following steps:

1. Get cough sounds after applying the cloud cough detection algorithm.
2. Start with N enrolment files and extract embedding using pretrained TitaNet. N can be a number, for example, between 5 to 20, preferably 10 to 15.

3. Compute reference embedding (median) from enrolment embeddings.

4. Compare similar files from the user cough audio dataset with the reference embedding.

5. Use files exceeding a predefined similarity score threshold for fine-tuning.

It will be understood that the TitaNet model is only one exemplary model that can be used. In general, other speaker verification algorithms may be used as well in a similar way. An exemplary cough audio dataset used for fine-tuning comprises the following parameters:

Number of targeted users: 205
Total number of files (minimum number of individual coughs): 19957
Audio duration: 7.85 hours
Average audio duration per file: 1.42 seconds

For testing, a manually labelled dataset as described below can be used:

Number of targeted users: 36
Total number of filed (minimum number of coughs): 7197
Audio duration: 2.8 hours
Average audio duration per file: 1.41 seconds

As illustrated in **Fig. 7,** the targeted cough detector 5 can comprise a machine learning model 52, which can be a speaker verification algorithm 52, e.g., the TitaNet Model. For each cough audio clip 51, the targeted cough detector 5 utilizing the machine learning algorithm 52 can generate a respective comparison parameter 53, which can for example be an embedding 53 of length 192. Each comparison parameter 53 can represent the cough pattern (i.e., signature) of the cougher which is unique to each individual.

The cough audio clip 51 can be of any duration, preferably between 1.0 - 2.0 seconds. Preferably, however, the cough audio clip 51 can be below a predefined maximum duration.

The comparison parameter 53 can be a 192-dimensional vector.

After generating the comparison parameter 53, further user-specific computation takes place. For the computation to take place, past cough audio clips and/or past comparison parameters 53 may be used to update a reference comparison parameter corresponding to the targeted user.

Preprocessing and training parameters of the speaker verification algorithm 52 can be changed to get optimum results for the targeted user cough detection.

In particular, significant changes can be made to the preprocessing module of the speaker verification algorithm 52 (e.g., TitaNet). Namely, a shorter fast Fourier transform (FFT) window and normalization can be used to capture the nature of explosive and short-lived cough sounds. The feature parameters, e.g., length of the FFT window, may be determined by feature engineering techniques. Also, data augmentation techniques can be used, e.g., Gaussian white noise addition and/or adding noises from external dataset.

The speaker verification algorithm 52 can be tuned for a predetermined number of epochs and the last checkpoint can be used as the final model for evaluation. The Adam optimizer and angular margin loss (introduced in, Koluguri, N. R. et al. "SpeakerNet: 1D Depth-wise Separable Convolutional Network for Text-Independent Speaker Recognition and Verification", (2020), https://arxiv.org/abs/2010.12653) can be used to optimize the weights.

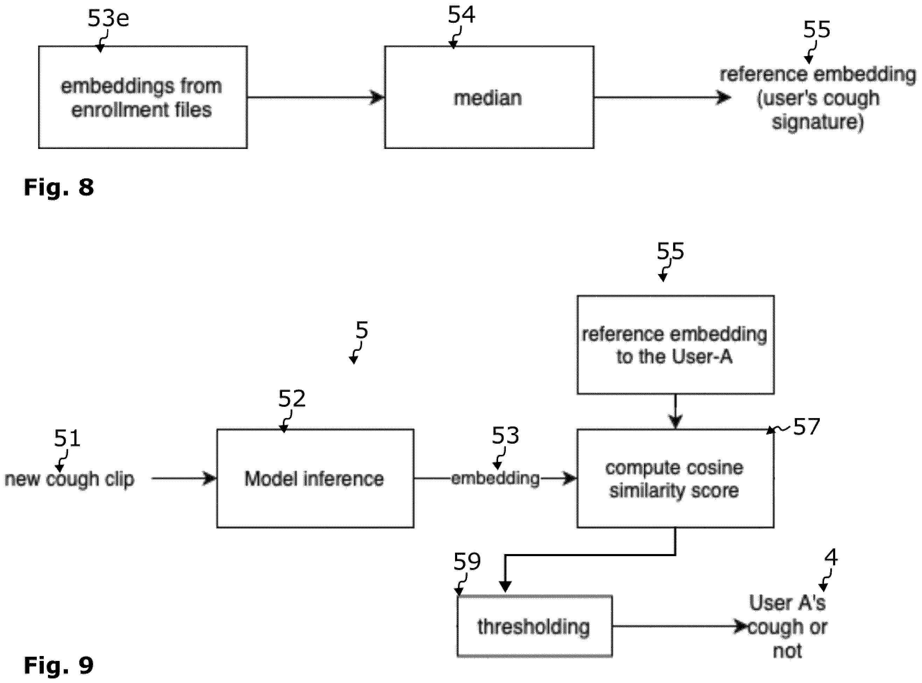

As illustrated in **Fig. 8,** a reference comparison parameter 55 can be determined by taking the median 54 of all comparison parameters 53e of the enrolment files.

As illustrated in **Fig. 9,** the reference comparison parameter 55, which can be a reference embedding 55, i.e., a cough signature of the targeted user, can be used as a ground truth to compare whether the cough belongs to the targeted user or not.

In particular, a cough clip 51 can be provided to the targeted cough detector 5, which based thereon can compute a comparison parameter 53 corresponding to the cough clip. The comparison parameter 53 can for example be an embedding 53 of the cough clip generated using a machine learning algorithm, such as, a speaker verification algorithm.

The comparison parameter 53 can be compared with the reference comparison parameter 54 and a similarity score 57 can be generated. For example, the cosine similarity can be used to compute the similarity score 57. The similarity score 57 can be compared with a similarity threshold to determine a result 4 indicative of whether the cough clip contains cough of the targeted user or not.

To account for potential changing cough pattern, the reference comparison parameter 55 can be updated, e.g., in certain intervals. One of the ways to achieve this is by updating the reference comparison parameter 55 every certain number of new cough audio clips 51 containing a cough of the targeted user. For example, for every 100 new targeted user's coughs, the reference comparison parameter can be recomputed.

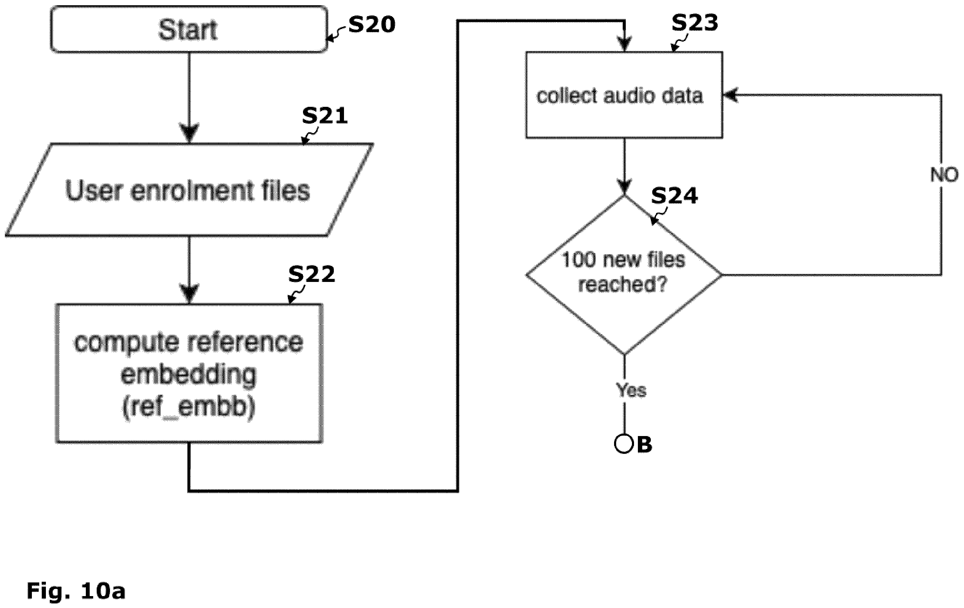

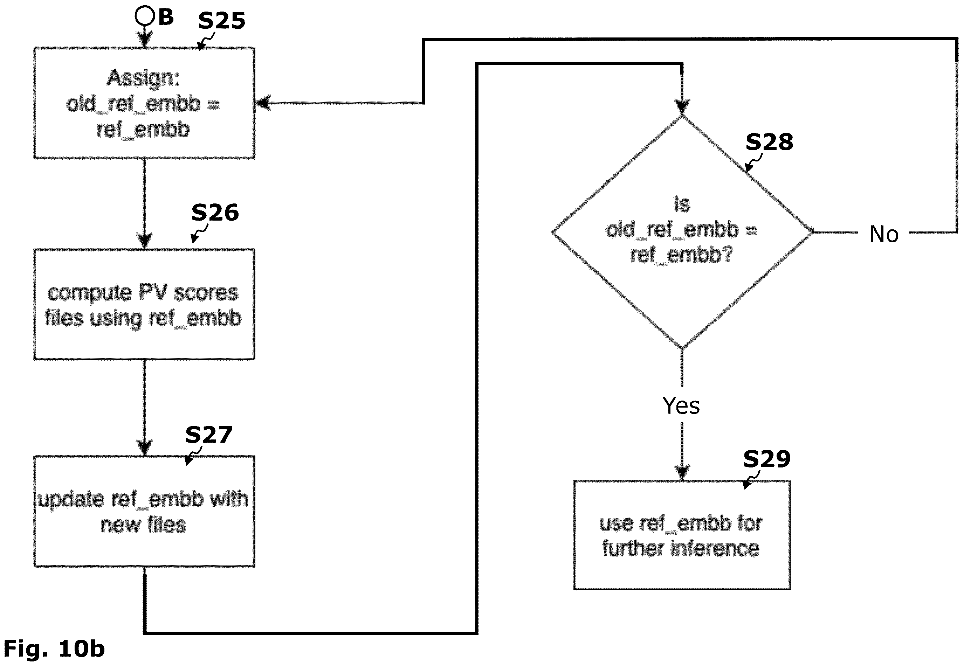

**Figs. 10a** and **10b** depict a flowchart of computing and updating a reference comparison parameter 55. It will be understood that Figs. 10a and 10b, each show a part of a flowchart depicting the logical flow of computing and updating a reference comparison parameter 55, said parts joinable at point B.

The process may start with S20. In S21, enrolment cough data can be obtained which can comprise enrolment cough audio clips. These can for example be recordings of the targeted user coughing (e.g., after being prompted to do so). Thus, the enrolment cough audio clips are obtained in S21 such that it can be known that they contain cough of the targeted user.

In S22 for based on the enrolment cough audio clips a reference embedding can be computed, e.g., as illustrated in Fig. 8 and discussed in relation thereto.

In S23, cough audio clips can be collected. These can form the cough audio dataset.

In S24 it can be checked whether a predetermined (e.g., 100) number of audio clips is reached. If no, the method returns to step S23 and continues collecting new cough audio clips. If yes, the method proceeds with S25 in Fig. 10b.

In S25, the reference comparison parameter 55 calculated in S22 can be stored in a secondary memory location.

In S26, similarity scores 57 for each of the cough audio clips collected in S23 can be computed using the reference comparison parameter 55 calculated in S22. The similarity scores 57 can be computed as illustrated in Fig. 9.

In S27 the reference comparison parameter 55 is updated using the cough audio clips collected in S23, preferably, only those cough audio clips collected in S23 for which the respective similarity score 57 is higher than an updating threshold. The updating can be done as illustrated in Fig. 8, e.g., by computing the median 54 of the comparison parameters 53 corresponding to cough audio clips collected in S23 for which the respective similarity score 57 is higher than an updating threshold.

In S28 it can be determined whether the value of the reference comparison parameter 55 has not changed, despite the update in S27. That is, in S28 it is checked whether the reference embedding before the update in S27 (i.e., the "old_ref embedding") is the same as the reference embedding before the update in S27. If not the same (i.e., the reference embedding is changed during the update), the method returns to S25. That is, the reference embedding has not yet converged. Thus, the reference comparison parameter is updated until there is no further change.

If yes (i.e., the reference embedding remains unchanged despite the update) the method proceeds with using the reference comparison parameter 55 for further inference. That is, the reference embedding has converged.

The targeted cough detector 5 can evaluate cough audio clips to determine whether they contain cough of the targeted user or not. The targeted cough detector 5 can output a respective 192-dimensional embedding 53 representation for each audio clip. Based on the embeddings, the cosine similarity can be used to verify whether cough sound belongs to the same user or not.

As explained, to determine whether a cough belongs to a user, a reference comparison parameter 53, i.e., a reference embedding, or a reference cough may be required to compare with. This can be challenging to obtain in practice, particularly for cough. Unlike speech sounds which can be produced by the user at any time of their convenience, cough is generally considered involuntary in nature. The voluntary (forced) cough might not represent the true nature of the user's cough. This poses challenge in using the enrolment coughs, which are voluntary in nature, as basis for computing the reference comparison parameter.

The enrolment cough, in particular the reference comparison parameter calculated based thereon, however, can be used to extract other cough within the training dataset which has high similarity. The audio clips within the training dataset comprising the highest similarity scores with the enrolment coughs can be iteratively extracted to formulate the reference comparison parameter - as discussed with reference to S25-S28. Therefore, the reference comparison parameter can be computed taking median of the comparison parameters of the extracted coughs. The reference comparison parameter can be used for comparison to a new cough sound, as in S29.

The reference comparison parameter can change with the nature of user's cough tone which can evolve with time. Hence, updating the reference comparison parameter at certain interval can be advantageous as it more accurate results may be achieved.

The evaluation process of the targeted cough detector can be summarized in following steps:

1. Compute reference embedding (ref_A) taking median of embeddings of the enrolment coughs.
2. Use the reference embedding to extract high confidence patient cough.
3. Update the reference embedding from extracted high confidence coughs.
4. Go to step 2 until there is no change in reference embedding (extracted files).
5. Use this reference embedding to compare similarity with future coughs.
6. Update reference embedding with new data as necessary.

Patient cough verification can be challenging in several aspects. First, due to difficulty in collecting data, there can be limited amount of data available for training large learning models. Moreover, to collect sufficient amount of involuntary cough data, the data collection operation needs to extend multiple days. During this data collection period, there might be temporary or permanent presence of other individual in the vicinity of the targeted user. Therefore, it cannot be ensured that the collected cough data belongs to the targeted user. The present invention can alleviate these issues to obtain reliable targeted user cough detection.

A goal of present invention can be to identify the intended user's cough data points from the rest. An aim can thus be achieving a high True Positive Rate (TPR) and a low False Positive Rate (FPR) for the targeted user, wherein

True Positive Rate (TPR) = True Positive / Positives
False Positive Rate (FPR) = False Positive / Negatives

Additionally, Equal Error Rate (EER), can be used, which is frequently used in verification tasks, to evaluate the target cough detector 5. The EER is the error when FPR = FNR (false negative rate). Lower the value of EER better is the model.

The test data used for evaluation comprises audio clips collected from 36 different targeted users which are manually labelled with assistance from the targeted cough detector 5.

Table IV shows the ability of using enrollment files to capture and predict the unseen test files. As shown in the flowchart in Figs. 10a and 10b, the reference comparison parameter is computed using all enrollment files (as in S22) and based thereon the similarity score for the next 100 files (max) as given in 2nd column of Table IV is computed (as in S26). If all enrolment files are used, the percentage of files as given in 3rd column of Table IV is captured. Here, a threshold of 0.64 is used. If the similarity score is higher than the threshold, the file is predicted as a true positive.

| Table IV | | |
|---|---|---|
| **Class** | **Test Files (unseen)** | **% Acceptance (TPR)** |
| 0 | 100 | 100.00% |
| 1 | 98 | 98.98% |
| 2 | 100 | 99.00% |
| 3 | 67 | 100.00% |
| 4 | 100 | 94.00% |
| 5 | 100 | 99.00% |
| 6 | 100 | 100.00% |
| 7 | 100 | 100.00% |
| 8 | 100 | 100.00% |
| 9 | 100 | 100.00% |
| 10 | 37 | 100.00% |
| 11 | 100 | 97.00% |
| 12 | 100 | 97.00% |
| 13 | 100 | 100.00% |
| 14 | 100 | 100.00% |
| 15 | 69 | 100.00% |
| 16 | 100 | 99.00% |
| 17 | 100 | 96.00% |
| 18 | 100 | 100.00% |
| 19 | 100 | 95.00% |
| 20 | 100 | 99.00% |
| 21 | 100 | 100.00% |
| 22 | 100 | 94.00% |
| 23 | 100 | 99.00% |

(continued)

| Table IV | | |
| --- | --- | --- |
| Class | Test Files (unseen) | % Acceptance (TPR) |
| 24 | 100 | 98.00% |
| 25 | 100 | 98.00% |
| 26 | 58 | 100.00% |
| 27 | 100 | 100.00% |
| 28 | 82 | 100.00% |
| 29 | 100 | 99.00% |
| 30 | 100 | 96.00% |
| 31 | 100 | 100.00% |
| 32 | 100 | 100.00% |
| 33 | 100 | 100.00% |
| 34 | 100 | 96.00% |
| 35 | 100 | 99.00% |

The above table shows that almost all of unseen files can be correctly predicted with the help of the reference embedding.

Next, these accepted files are used to compute a new reference embedding (as in S27) which can be used to infer the next 200 (maximum) files. Table V summarizes the unseen files taken for evaluation. Whether these files are completely unique(unseen) is denoted by the third column in Table V. If the value in 3rd column in yes, it is completely unique, otherwise the files are same as represented in above table which is used for computation of the reference embedding.

| Table V | | |
| --- | --- | --- |
| Class | Test Files taken | Unique (unseen) Files |
| 0 | 200 | yes |
| 1 | 98 | no |
| 2 | 200 | yes |
| 3 | 67 | no |
| 4 | 20 | yes |
| 5 | 56 | yes |
| 6 | 52 | yes |
| 7 | 200 | yes |
| 8 | 200 | yes |
| 9 | 90 | yes |
| 10 | 37 | no |
| 11 | 200 | yes |
| 12 | 200 | yes |
| 13 | 68 | yes |
| 14 | 24 | yes |
| 15 | 69 | no |
| 16 | 200 | yes |

(continued)

| Table V | | |
|---|---|---|
| Class | Test Files taken | Unique (unseen) Files |
| 17 | 200 | yes |
| 18 | 200 | yes |
| 19 | 188 | yes |
| 20 | 128 | yes |
| 21 | 200 | yes |
| 22 | 155 | yes |
| 23 | 105 | yes |
| 24 | 200 | yes |
| 25 | 200 | yes |
| 26 | 58 | no |
| 27 | 200 | yes |
| 28 | 82 | no |
| 29 | 200 | yes |
| 30 | 129 | yes |
| 31 | 200 | yes |
| 32 | 141 | yes |
| 33 | 200 | yes |
| 34 | 88 | yes |
| 35 | 36 | yes |
| Total: | 4891 | yes: 30, no:6 |

To test the ability of the targeted cough detector to separate out coughs of undesired users, the FPR can be computed for each test user against all other user's cough, as depicted in Table VII.

| Table VII | | | | |
|---|---|---|---|---|
| CLASS | without reference embedding update (THRESH=0.65) | | With reference embedding update (THRESH=0.66) *threshold could be high for low FPR value | |
| | TPR | FPR | TPR | FPR |
| 0 | 99.50% | 3.22% | 100.00% | 2.73% |
| 1 | 97.96% | 6.41% | 96.94% | 4.90% |
| 2 | 100.00% | 3.30% | 98.50% | 3.35% |
| 3 | 100.00% | 4.75% | 100.00% | 3.54% |
| 4 | 100.00% | 2.20% | 100.00% | 1.19% |
| 5 | 100.00% | 1.03% | 96.43% | 0.58% |
| 6 | 100.00% | 4.96% | 98.08% | 3.45% |
| 7 | 100.00% | 3.90% | 100.00% | 2.56% |
| 8 | 99.50% | 2.62% | 99.50% | 1.81% |
| 9 | 97.78% | 5.29% | 96.67% | 4.52% |
| 10 | 100.00% | 1.34% | 100.00% | 0.70% |

(continued)

| Table VII | | | | |
|---|---|---|---|---|
| CLASS | without reference embedding update (THRESH=0.65) | | With reference embedding update (THRESH=0.66) *threshold could be high for low FPR value | |
| | TPR | FPR | TPR | FPR |
| 11 | 93.50% | 5.59% | 95.50% | 2.22% |
| 12 | 95.50% | 2.81% | 98.50% | 2.28% |
| 13 | 100.00% | 5.31% | 100.00% | 3.34% |
| 14 | 100.00% | 3.62% | 100.00% | 1.66% |
| 15 | 100.00% | 4.02% | 100.00% | 3.05% |
| 16 | 100.00% | 7.48% | 99.50% | 5.52% |
| 17 | 95.00% | 3.28% | 94.00% | 1.96% |
| 18 | 99.00% | 1.73% | 99.50% | 1.62% |
| 19 | 95.74% | 3.40% | 94.68% | 3.00% |
| 20 | 100.00% | 3.82% | 100.00% | 1.30% |
| 21 | 91.50% | 2.13% | 97.00% | 1.32% |
| 22 | 87.10% | 7.26% | 92.26% | 5.62% |
| 23 | 96.19% | 3.32% | 90.48% | 2.88% |
| 24 | 97.50% | 2.22% | 99.50% | 1.22% |
| 25 | 95.50% | 5.88% | 99.50% | 4.18% |
| 26 | 100.00% | 3.15% | 100.00% | 2.48% |
| 27 | 98.50% | 7.65% | 98.00% | 6.22% |
| 28 | 100.00% | 3.45% | 100.00% | 2.62% |
| 29 | 96.00% | 5.09% | 98.50% | 4.86% |
| 30 | 80.62% | 5.65% | 92.25% | 2.92% |
| 31 | 99.00% | 4.16% | 98.50% | 3.43% |
| 32 | 100.00% | 4.40% | 98.58% | 3.62% |
| 33 | 96.00% | 2.05% | 98.00% | 1.34% |
| 34 | 97.73% | 4.85% | 100.00% | 2.50% |
| 35 | 100.00% | 5.58% | 100.00% | 4.61% |
| **Average** | **97.48%** | **4.08%** | 98.89% | 3.97% |
| **Total (without class info.)** | **97%** | **4%** | 98% | 3% |

Table VIII provides the value of TPR when FPR is fixed at 1.0%.

| Table VIII | |
|---|---|
| **TPR (when FPR=1.0%)** | |
| **Without updated reference embedding** | **Updated reference embedding** |
| 89.3% | 93.9% |

Tabel IX compares the result without updated reference embedding vs updated reference embedding. The patient-wise average EER is computed which is the average value of EER of all users and is given as:

$$\text{Patient-wise average EER} = \text{sum EER of all users} / \text{num of users}.$$

Additionally, total EER is computed which is the value computed over all test files: Total EER = EER computed over all test files (not taking individual classes in account)

| Table IX | | |
|---|---|---|
| METRICS | Without updated reference embedding | Updated reference embedding |
| **Patient-wise average EER** | 2.86% | 2.09% |
| **Total EER** | 3.59% | 2.49% |

The Patient-wise average EER gives the expected value of user EER while the total EER gives the sense of how good the target cough detector can be in discerning different users.

The data thus indicates that the targeted cough detector is able to detect targeted user's cough reliably. Additionally, updating the reference embedding routinely can help to boost the performance of the cough detection.

Various cough metrics 7 can be generated that can be beneficial for diagnosis of various disease where cough is a symptom. A quantitative measurement as well as qualitative features of cough can be generated.

While the present invention has been described with reference to particular embodiments, it is to be understood that these embodiments do not limit the scope of the invention, but merely serve to illustrate the invention.

Whenever a relative term, such as "about", "substantially" or "approximately" is used in this specification, such a term should also be construed to also include the exact term. That is, e.g., "substantially straight" should be construed to also include "(exactly) straight".

Whenever steps were recited in the above or also in the appended claims, it should be noted that the order in which the steps are recited in this text may be accidental. That is, unless otherwise specified or unless clear to the skilled person, the order in which steps are recited may be accidental. That is, when the present document states, e.g., that a method comprises steps (A) and (B), this does not necessarily mean that step (A) precedes step (B), but it is also possible that step (A) is performed (at least partly) simultaneously with step (B) or that step (B) precedes step (A). Furthermore, when a step (X) is said to precede another step (Z), this does not imply that there is no step between steps (X) and (Z). That is, step (X) preceding step (Z) encompasses the situation that step (X) is performed directly before step (Z), but also the situation that (X) is performed before one or more steps (Y1), ..., followed by step (Z). Corresponding considerations apply when terms like "after" or "before" are used.

While in the above, preferred embodiments have been described with reference to the accompanying drawings, the skilled person will understand that these embodiments were provided for illustrative purpose only and should by no means be construed to limit the scope of the present invention, which is defined by the claims.

**Claims**

1. A method to detect cough of a targeted user, the method comprising:

   processing a cough audio clip with a targeted cough detector and
   generating based thereon a result indicative of whether the cough audio clip contains a cough sound of the targeted user;
   wherein the method comprises obtaining source audio data, wherein the source audio data comprise the cough audio clip; and
   wherein the method comprises pre-processing the source audio data with a cough detector to obtain cough audio data, wherein the cough audio data comprise the cough audio clip.

2. The method according to the preceding claim, wherein the pre-processing is performed such that the cough audio data contain less non-cough sounds than the source audio data.

3. The method according to any of the preceding claims, wherein the source audio data is partitioned in audio segments and
   wherein the pre-processing comprises determining for each of the audio segments whether to include it in the cough audio data;
   wherein determining for each of the audio segments whether to include it in the cough audio data comprises including in the cough audio data one or more of the audio segments for which the cough detector determines a positive indication on whether the audio segment contains a cough sound.

4. The method according to the preceding claim, wherein determining for each audio segment whether to include it in the cough audio data comprises filtering out one or more of the audio segments for which the cough detector determines a positive indication on whether the audio segment contains a non-cough sound.

5. The method according to any of the preceding claims, wherein the cough detector comprises multiple stage detectors arranged sequentially such that each subsequent stage detector receives as input the output of a preceding stage detector;
wherein each stage detector determines for each audio segment that it receives whether to include it in the cough audio data.

6. The method according to any of the preceding claims, wherein the pre-processing comprises

generating potentially cough audio data based on the source audio data and
generating the cough audio data based on the potentially cough data;
wherein the potentially cough data contains less non-cough sounds than the source audio data;
wherein the cough audio data contains less non-cough sounds than the potentially cough data;
wherein the cough detector comprises an edge detector and wherein generating the potentially cough audio data based on the source audio data is performed by the edge detector;
wherein the cough detector comprises a cloud detector and wherein generating the cough audio data based on the potentially cough audio data is performed by the cloud detector and
wherein the method comprises transmitting the cough audio data from the cloud detector to the targeted cough detector.

7. The method according to the preceding claim, wherein the cloud detector comprises more computational resources than the edge detector.

8. The method according to any of the preceding claims and with the features of claim 3, wherein the cough audio clip comprises one or more of the audio segments included in the cough audio data.

9. The method according to any of the preceding claims, wherein the processing comprises

computing a comparison parameter corresponding to the cough audio clip,
comparing the comparison parameter with a reference comparison parameter corresponding to the targeted user,
generating the result based on the comparison.

10. The method according to the preceding claim, wherein the method comprises initializing the reference comparison parameter and wherein said initializing comprises generating the reference comparison parameter based on enrolment cough data comprising cough recordings of the targeted user.

11. The method according to any of the 2 preceding claims, wherein the method comprises updating the reference comparison parameter and
wherein updating the reference comparison parameter is based on past cough audio clips, wherein the past cough audio clips are generated prior to the cough audio clip.

12. The method according to any of the 3 preceding claims, wherein the method comprises computing a similarity score between the comparison parameter and the reference comparison parameter and
wherein each of the past cough audio clips used for updating the reference comparison parameter comprise a respective comparison parameter, the similarity score of which is higher than an updating threshold.

13. A system for detecting cough of a targeted user, the system comprising:

a targeted cough detector configured to process a cough audio clip and to generate based thereon a result indicative of whether the cough audio clip contains a cough sound of the targeted user;
wherein the system is configured to obtain source audio data, wherein the source audio data comprise the cough audio clip;
wherein the system comprises a cough detector configured to pre-process the source audio data to obtain cough audio data, wherein the cough audio data comprise the cough audio clip.

14. The system according to the preceding claim, wherein the cough detector is configured to

generate potentially cough audio data based on the source audio data and
generate the cough audio data based on the potentially cough data;
wherein the cough detector comprises an edge detector configured to generate the potentially cough audio data based on the source audio data;
wherein the cough detector comprises a cloud detector configured to generate the cough audio data based on the potentially cough audio data.

15. A computer program product comprising instructions which, when the program is executed by the system according to claim 13 or 14, cause the system to carry out the method according to any of the preceding claims 1 to 12.

EP 4 674 344 A1

**1**

audio recorder

**11**

**3**

Pre-filter

**15**

**5**

Targeted Cough Detection

**7**

cough metrics

**Fig. 1**

**10** **31**

**1**

Audio recorder

**11**

edge cough detector

**12**

**32** **20** **15** **5**

cloud cough detector

Patient Verification

**7**

cough metrics

**Fig. 2**

**Fig. 3**

**Fig. 4**

EP 4 674 344 A1

**S1** audio input (30-60s)

**102** audio preparator

**S2** overlapping chunk of 1 second duration

**S3** normalize

**103** feature engineering

**S4** melspectogram

○**A**

**11a**

**8a** **8b** **8c** **8d**

each chunk has highest amplitude of value 1

calculate log mel spectrogram

**Fig. 5a**

EP 4 674 344 A1

A

S5

Model Inference

use TFLite model (MobileNetV2)

S6

post-processing

decide cough/non-cough based on the prediction probability

S7

cough locations

get cough locations with reference to input audio

**Fig. 5b**

EP 4 674 344 A1

202

32

203

12

audio file → | audio preparator | → | feature generator |

204

206

| Transformer (PaSST) | → | Post-processing | → cough or not 15

**Fig. 6**

52

51

cough clip → | TItaNet Model | → embeddings

53

**Fig. 7**

EP 4 674 344 A1

**Fig. 8**

**Fig. 9**

Fig. 10a

S23 — collect audio data

S24 — 100 new files reached?

NO

Yes — B

S20 — Start

S21 — User enrolment files

S22 — compute reference embedding (ref_embb)

**Fig. 10b**

EP 4 674 344 A1

Europäisches
Patentamt

European
Patent Office

Office européen
des brevets

# EUROPEAN SEARCH REPORT

**Application Number**

EP 24 18 5753

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/388006 A1 (HARRIS BASIL M [US] ET AL) 26 December 2019 (2019-12-26) * paragraph [0055] - paragraph [0059] * ----- | 1-15 | INV. A61B5/08 A61B7/00 |
| X | US 2022/130415 A1 (GARRISON JACOB [US] ET AL) 28 April 2022 (2022-04-28) * claim 1 * ----- | 1,13,15 | |
| X | US 12 004 851 B1 (CIRCO IULIAN-ALEXANDRU [AT] ET AL) 11 June 2024 (2024-06-11) * claim 1 * ----- | 1,13,15 | |

|  | TECHNICAL FIELDS SEARCHED (IPC) |
|---|---|
|  | A61B |

The present search report has been drawn up for all claims

| Place of search | Date of completion of the search | Examiner |
|---|---|---|
| Berlin | 17 December 2024 | Kyriakides, Leonidas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 674 344 A1**

## ANNEX TO THE EUROPEAN SEARCH REPORT
## ON EUROPEAN PATENT APPLICATION NO.

EP 24 18 5753

This annex lists the patent family members relating to the patent documents cited in the above-mentioned European search report.
The members are as contained in the European Patent Office EDP file on
The European Patent Office is in no way liable for these particulars which are merely given for the purpose of information.

17-12-2024

| Patent document cited in search report | | Publication date | Patent family member(s) | | Publication date |
|---|---|---|---|---|---|
| US 2019388006 | A1 | 26-12-2019 | US | 2019388006 A1 | 26-12-2019 |
| | | | WO | 2018107008 A1 | 14-06-2018 |
| US 2022130415 | A1 | 28-04-2022 | US | 2022130415 A1 | 28-04-2022 |
| | | | US | 2024161769 A1 | 16-05-2024 |
| US 12004851 | B1 | 11-06-2024 | US | 12004851 B1 | 11-06-2024 |
| | | | WO | 2024163390 A1 | 08-08-2024 |

EPO FORM P0459

For more details about this annex : see Official Journal of the European Patent Office, No. 12/82

45

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20220130415 A1

- US 20220409089 A1

**Non-patent literature cited in the description**

- **C. -Y. WU** ; **R. MANMATHA** ; **A. J. SMOLA** ; **P. KRÄHENBÜHL**. Sampling Matters in Deep Embedding Learning. *2017 IEEE International Conference on Computer Vision (ICCV), Venice, Italy*, 2017, 2859-2867
- **F. SCHROFF** ; **D. KALENICHENKO** ; **J. PHILBIN**. FaceNet: A unified embedding for face recognition and clustering. *2015 IEEE Conference on Computer Vision and Pattern Recognition (CVPR), Boston, MA, USA*, 2015, 815-823
- **SANDLER, MARK et al.** *MobileNetV2: Inverted Residuals and Linear Bottlenecks*, 2019, https://doi.org/10.48550/arXiv.1801.04381
- **FONSECA, E. et al.** *FSD50K: An Open Dataset of Human-Labeled Sound Events*, 2022, https://arxiv.org/abs/2010.00475
- **ORLANDIC L. et al.** *The COUGHVID crowdsourcing dataset, a corpus for the study of large-scale cough analysis algorithms*, 2021, https://www.nature.com/articles/s41597-021-00937-4
- **SON CHUNG, J. et al.** *VoxCeleb2: Deep Speaker Recognition*, 2018, https://arxiv.org/abs/1806.05622

- **SNYDER, D.** *MUSAN: A Music, Speech, and Noise Corpus*, 2015, https://arxiv.org/abs/1510.08484
- **TRAER, J et al.** *Statistics of natural reverberation enable perceptual separation of sound and space*, 2016, https://mcdermottlab.mit.edu/Reverb/IR Survey.html
- **KINGMA, D. P. et al.** *Adam: A Method for Stochastic Optimization*, 2017, https://arxiv.org/abs/1412.6980
- **KOUTINI, K. et al.** *Efficient Training of Audio Transformers with Patchout*, 2022, https://arxiv.org/abs/2110.05069
- **KOLUGURI, N. R. et al.** *TitaNet: Neural Model for speaker representation with 1D Depth-wise separable convolutions and global context*, 2021, https://arxiv.org/abs/2110.04410
- **KOLUGURI, N. R. et al.** *SpeakerNet: 1D Depth-wise Separable Convolutional Network for Text-Independent Speaker Recognition and Verification*, 2020, https://arxiv.org/abs/2010.12653